# EUROPEAN PATENT APPLICATION

(11) **EP 1 886 679 A2**
(43) Date of publication of application: **13.02.2008**
(21) Application number: 07075522.8
(22) Date of filing: 03.06.1996
(51) Int. Cl.: A61K 31/20, A61K 31/23, A61K 36/02, A61K 36/06

(54) **Methods for controlling highly unsaturated fatty acid content in various tissues**

(30) Priority: 07.06.1995 US 479809
(62) Divisional of application: 96921263.8
(71) Applicant: MARTEK BIOSCIENCES CORPORATION, Columbia, MD 21228 (US)
(72) Inventor: Kyle, David J., Catonsville MA 21228 (US); Linsert, Henry, Alexandria VA 22306 (US)
(74) Representative: Mercer, Christopher Paul

(57) **Abstract**

A method of treating a neurological disorder comprises administering to a person affected from such a disorder a microbial oil comprising DHA, a microbial oil comprising ARA or a combination of DHA and ARA oils in an amount sufficient to elevate the levels of circulating DHA and/or ARA in the person's blood to at least normal levels.

## Description

### Field of the Invention

This invention relates to methods of treating diseases associated with deficiencies in highly unsaturated fatty acids (HUFA), such as neurological diseases, cardiac diseases, etc., by administering therapeutic compounds to supplement HUFA levels in the patient. In particular, this invention relates to a method of treating neurological disorders, including certain neurodegenerative diseases and psychiatric disorders, by administering a composition comprising a therapeutically effective amount of a single cell microbial oil comprising docosahexaenoic acid (DHA), a single cell oil comprising arachidonic acid (ARA) or a combination of DHA- and ARA-containing oils, to a person in need of such treatment. The oils can be administered as a pharmaceutical composition, as a dietary supplement, or in the form of a food product by replacing a portion of the vegetable oil or fat thereon.

### BACKGROUND OF THE INVENTION

The human brain and other neural tissues are highly enriched in long chain polyunsaturated fatty acids which are thought to play an important role in modulating the structure, fluidity and function of the cell membranes of these tissues. Arachidonic acid (hereafter referred to as ARA) is a long chain polyunsaturated fatty aid of the ω-6 class (5, 8, 11, 14-eicosatetraenoic acid. 20:4ω-6), and is the most abundant C₂₀ polyunsaturated fatty acid in the human body. In addition to its primary role as a structural lipid, ARA also is the direct precursor for a number of circulating eicosenoids such as prostaglandin E₂ (PGE₂) prostacyclin I₂ (PGI₂) thromboxane A₂ (TₓA₂), and leukotirenes B₄ (LTB₄) and C₄ (LTC₄). The eicosenoids exhibit regulatory effects on lipoprotein metabolism, blood rheology, vascular tone, leucocyte function and platelet activation. In humans ARA is not synthesized *de novo,* but it can be synthesized by the elongation and desaturation of linoleic acid, an essential fatty acid which must be obtained from the diet.

Docosahexanoic acid (4, 7, 10, 13, 16, 19-docosahexanoic acid 22:6ω-3) (hereinafter referred to as DHA) is the most abundant of the fatty acids of the structural components of grey matter of the human brain and other neural tissues. DHA cannot be synthesized *de novo* in humans, but there is some evidence that this ω-3 fatty acid can be synthesized by some cell types, such as astrocytes, if the appropriate long chain polyunsaturated fatty acids are provided in the diet. S. Moore, et al., 1991, J. Neurochem., 56:518-524. Most of the DHA found in the brain and retina cell membranes is believed to be obtained from dietary sources.

The importance of providing polyunsaturated fatty acids during a period of rapid brain development to prevent irreparable damage to brain cells is well known in the art. Human infants appear to have a particularly poor ability to synthesize DHA, but any deficiencies can be compensated for by feeding infants human breast milk, which is a rich source of essential fatty acids, particularly DHA and ARA. Sanders, et al, 1978, J. Clin. Nutr. , 31:805-813. Recent studies comparing the performance on standard intelligence tests of children who were fed breast milk as babies to children who were fed commercial infant formulas as babies have suggested a dose response relationship between the proportion of mother's milk in the diet and subsequent IQ. Lucas, et al., 1992, Lancet, 339:261-264. These studies suggest that dietary intervention therapy can effect the levels of DHA available for structural development of the nervous system.

It has been observed that DHA levels in two major classes of phospholipid, phosphatidylethanolamine and phosphatidylcholine, are significantly reduced in the brain tissues of patients with Alzheimer's disease. Control samples taken from patients of advanced age, having no clinical manifestations of dementia or other disorders who showed no significant changes in the fatty acid composition of these two classes of phospholipids. These results suggest that the alterations in DHA concentrations in the brain tissue of Alzheimer's patients are not the result of normal aging, but are specific for the pathological mechanisms involved in this neurodegenerative disease. Soderberg, et al., 1991, Lipids, 26:421-425.

Peroxisomal disorders are a group of degenerative neurological disorders characterized by increased levels of very long chain fatty acids, resulting from an impaired capacity of the effected individuals for degrading these fatty acids. These disorders are related in that they all appear to result from some defect localized in the subcellular organelles known as peroxisomes. Gordon, N., 1987, Brain Development, 9:571-575. These peroxisomal disorders have been classified into three groups based on the extent of the loss of peroxisomal functions found in a particular disease. Theil, A.C., 1992, European Journal of Pediatrics, 151:117-120.

The group 1 peroxisomal disorders are characterized by a virtually complete loss of peroxisomes and peroxisomal functions. These disorders include Zellwebger's syndrome, neonatal adrenoleukodystrophy, infantile Refsum disease and hyperpepecolic acidemia. The group 2 disorders are characterized by the loss of multiple peroxisomal functions and include Rhizomelic chondrodysplasia punctata and Zellweger-ike syndrome. The group 3 disorders are characterized by the loss of only a single peroxisomal function and include adrenoleukodystrophy, adrenomyeloneuropathy, acyl-CoA oxidase deficiency, bifunctional protein deficiency, thiolase deficiency, hyperoxaluria type I, acatalasaemia and adult Refsum disease. Clinical presentation of patients with peroxisomal disorders shows a wide divergence in phenotypic expression which varies significantly depending upon the patient's age. However, in all patients neurological functions are progressively impaired, which often leads to deterioration of the autonomic functions and death at an early age.

Recent studies of the polyunsaturated fatty acid composition of tissues in patients with peroxisomal disorders have shown that, even though the total amount of fatty acids in these tissues was normal, there are significant changes in the fatty acid composition of the patient's tissues. These patients have a significant decrease in the total amount of DHA and ARA in their serum lipid compositions. Serum plasmalogen levels are also depressed.

Usher's syndrome is an autosomal recessive genetic disorder which is associated with the degeneration of visual cells, causing retinitis pigmentosum. The visual cells contain extremely large quantities of DHA esterified in the phospholipids of the photoreceptor membranes which make up the outer segments of the visual cells. Bazan and coworkers recently have found that the plasma phospholipids of Usher's patients contain significantly less DHA and ARA than the plasma phospholipids of unaffected individuals. Bazan, et al. , 1986, Biochem. Biophys. Res. Comm. , 141:600-604.

In addition researchers have found that patients suffering from other clinical conditions, such as senile dementia, diabetes-induced neuropathy, multiple sclerosis, schizophrenia and neuropathies associated with high doses of heavy metals such as lead, aluminum, and mercury also frequently have levels of DHA and/or ARA in their serum lipids which are significantly depressed in comparison to the levels found in healthy persons. For example, recent studies have established a correlation between alternations in the levels of esterification of ARA into the phospholipids of platelets and the presence of schizoaffective disorders in patients. Demisch, et al., 1992, Prostaglandins Leukot. Essent. Fatty Acids, 46:47-52. Evidence of abnormal essential fatty acid biochemistry int he plasma phospholipids of patients with schizophrenia also has been reported. Horrobin, D.F., 1992, Prostaglandins Leukot. Essent. Fatty Acids, 46:71-77.

Although researchers have made some progress in understanding neurodegenerative disorders such as Alzheimer's disease and various peroxisomal disorders, effective means of treating these disorders have remained elusive. Likewise, there has been a lack of progress in the development of effective therapeutic drugs to treat schizophrenia and other neurological disorders disclosed above.

Marine oils, which are extracted from fish and have high content of the omega-3 fatty acids eicosapentaenoic (EPA) and docosaheacaenoic (DHA), have been shown to lower plasma triglycerides and inhibit platelet aggregation, as well as favorably altering monocyte function (Davidson, et al., 1979, "Marine Lipids and Atherosclerosis: A Review," Cardiovascular Reviews & Report, 7:5). Based on such observations, there has been considerable clinical interest in the use of marine oils as part of preventive therapy for atherosclerosis (Weiner, et al., 1986, "Inhibition of Atherosclerosis by Cod Liver Oil in a Hyperlipidemic Swine Model," N. Eng. J. Med., 314:841-845). Fujita, et al., U.K. Published Application No. 2 098 065, reported that compositions containing only one of the ω-3 fatty acids EPA or DHA (as ethyl esters) will have the same effect on platelet agglutination. In other words, EPA and DHA were found to be equivalent in their effects, and ethyl esters of either fatty acid were recommended as an alternative for fish oil due to reduction in objectionable taste as a result of extensive purification necessary to isolate the individual fatty acid.

However, it has also been reported that marine oil capsules rich in both EPA and DHA raise LDL-cholesterol levels (the fraction of serum cholesterol that is the most atherogenic), particularly in patients with Type IIb and IV dyslipidemia (Davids, et al., 1991, "Therapy for the Treatment of Hyperlipidemia," Archives of Internal Medicine, 151:1732-1740; Harris, et al., 1988, "Effects of a Low Saturated Fat, Low Cholesterol Fish Oil Supplement in Hypertriglyceridemia Subjects," Ann. Intern. Med., 109:465-470). In view of the effect of marine oils on cholesterol metabolism, clinical enthusiasm for the use of marine oil capsules has been dampened. Accordingly, there is a need for therapy having the beneficial circulatory effects of marine oils without the side effects of raising cholesterol.

### SUMMARY OF THE INVENTION

Accordingly, it is an object of the present invention to provide a method for treating neurological disorders, in which the serum, tissue or membrane levels of the essential fatty acids DHA and ARA are affected.

It is a further object of this invention to provide a method for lowering triglyceride content in plasma of a patient without the side effect of raising plasma cholesterol associated with marine oils.

It is yet another object of this invention to provide a method for correcting lipid imbalance in a patient having abnormal serum lipid profile by supplying a lipid supplement comprising highly unsaturated fatty acids (HUFA).

These and other objects are met by one or more of the embodiments of this invention. In one embodiment, this invention provides a method of increasing the level of highly unsaturated fatty acid (HUFA) residues in one or more tissues of a patient having a disease characterized by a HLTFA deficiency-associated pathology, by administering a therapeutic composition which includes oil containing HUFA residues to the patient in an amount effective to raise the level of HUFA residues in the patient's tissue. Preferably the level of HUFA residues in the patient's tissue is raised to normal. Diseases which may be treated by the method of this invention include neurological disorders, such as Alzheimer's disease, Huntington's disease, schizophrenia, diabetic neuropathy, heavy metal toxicity, and peroxisomal disorders including Zellweger's syndrome, neonatal adrenoleukodystrophy, infantile Refsum disease, hyperpepecolic acidemia, Rhizomelic chondrodysplasia punctata, Zellweger-like syndrome, adrenoleukodystrophy, adrenomyeloneuropathy, acyl-CoA oxidase deficiency, bifunctional protein deficiency, thiolase deficiency, hyperoxaluria type I, acatalasaemia and adult Refsum disease, as well as multiple sclerosis, cerebral palsy, amyotrophic lateral sclerosis, phenylketonuria and cystic fibrosis. In a preferred embodiment, the HUFA which is deficient in the patient, and which is enriched in the therapeutic composition, is DHA, the DHA level preferably being normalized in neuronal or brain tissue.

In another embodiment, this invention provides a method of lowering triglyceride content in plasma of a patient by administering an oil enriched in DHA to a patient in an amount effective to lower the level of plasma triglycerides in the patient. In a preferred embodiment, the oil enriched in DHA is administered in an amount effective to raise the level of circulating DHA in the patient's blood to at least about 50% above the normal level of plasma DHA, more preferably to twice the normal level. Alternatively, the amount of oil enriched in DHA is effective to raise the level of circulating DHA to within the range of about 15 to 100 µg of DHA per ml of plasma.

In yet another embodiment, this invention provides a method for correcting lipid imbalance in a patient having an abnormal lipid profile by supplying to the patient a lipid supplement comprising highly unsaturated fatty acids (HUFA), the lipid supplement being prepared by computing a "deviation" for each HUFA, which is the difference between normal tissue level of each HUFA and the level of each HUFA in the tissue of the patient, and preparing a therapeutic supplement by combining a plurality of individual HUFA-containing oils, at least one of which contains one HUFA but is substantially free of all other HUFAs, where the relative amounts of the individual oils being combined are such that the amount of each HUFA in the therapeutic supplement is approximately proportional to its computed deviation. One of the individual HUFA-containing oils may be fish oil. An effective amount of the therapeutic supplement is then administered to the patient to increase the tissue content of those HUFA which deviate most greatly from normal, preferably in an amount sufficient to restore normal tissue lipid profile to the patient. In a preferred embodiment, the tissue for which HUFA deviations are computed is blood, and more preferably, administration of the therapeutic supplement does not raise the level of any HUFA in the patient's serum above the normal serum level.

In still another embodiment, this invention provides a method of enhancing the ability of excitable cell membranes to sustain a chemical gradient of calcium concentration across the cell membranes by treating the cells with an amount of oil enriched in DHA which is effective to raise the level of DHA residues in the cell membranes, particularly for neurological cells or cardiac muscle cells.

This invention relates to a method of treating a patient suffering from a neurological disease, which comprises administering to the patient an effective amount of the fatty acids DHA or ARA, or a mixture of DHA and ARA. These fatty acids are administered in the form of oils in which DHA and ARA are provided as natural complex lipids, preferably in the form of triglycerides. The neurological diseases to be treated include the group of diseases classified as peroxisomal diseases, Alzheimer's disease, and Usher's syndrome, senile demential, diabetes-induced neuropathy, multiple sclerosis, schizophrenia and neuropathies associated with high doses of heavy metals such as lead, aluminum, and mercury, as well as other neurodegenerative diseases in which the serum, tissue or membrane concentrations of DHA or ARA are significantly affected in comparison to the DHA and ARA concentrations found in normal individuals.

The invention also relates to pharmaceutical compositions containing DHA or ARA or to a composition containing both DHA and ARA which provide therapeutically effective amounts of these ω-3 and ω-6 fatty acids. Administration of these compositions provides prophylactic, as well as therapeutic, treatment of patients diagnosed with neurodegenerative disorders or with other DHA and ARA deficiency-related disorders such as schizophrenia. These methods of treatment and compositions also provide a prophylactic treatment for individuals who are at risk for developing one of these neurological or other disorders.

### BRIEF DESCRIPTION OF THE DRAWINGS

Figure 1 is a graph of the accumulation of *C. cohnii* biomass in a 350 liter (gross volume) semi-pilot production scale fermentation.
Figure 2 is a graph of the (gross volume) accumulation of cohnii biomass in a 15,000 liter production scale fermentation for the production of DHA-containing microbial oil.
Figure 3 is a graph of the accumulation of *Mortierella alpina* in a 7,500 liter (gross volume) production scale fermentation for the production of ARA containing microbial oil.
Figure 4 shows the correlation between hippocampal DHA content and the relative distance (% of total distance) in the wrong annulus during water maze training. X-axis = DHA content, Y-axis = % distance in wrong annulus, • = aged rats, and ◆ = young rats.

### DETAILED DESCRIPTION OF THE INVENTION

In accordance with the present invention, a method is provided for treating diseases associated with deficiencies in highly unsaturated fatty acids (HUFA), such as neurological diseases, cardiac diseases, etc., by administering therapeutic compositions to supplement HUFA levels in the patient. In particular embodiments, a method for treating a neurological disorder or other disease associated with depressed levels of DHA and/or ARA in the blood or tissues, or with neural or muscle cell membranes that are "leaky" to calcium, is provided which comprises administering to a person suffering from such a disorder a microbial oil comprising DHA, a microbial oil comprising ARA or a combination thereof. These neurological disorders include neurodegenerative disorders and certain psychiatric disorders such as schizophrenia, in which the serum, tissue or membrane levels of the essential fatty acids DHA and ARA are affected. The DHA and ARA are in the from of natural complex lipids. Preferably, the DHA and ARA are in the form of triglycerides, although they also may be in the form of phospholipids. They are obtained as single cell microbial oils by the cultivation of DHA-producing microorganisms or ARA-producing organisms under oil-producing conditions.

According to the preferred embodiments of the present invention, microorganisms capable of producing a single cell microbial oil containing DHA or ARA are cultivated in a fermenter in a nutrient solution capable of supporting the growth of such organisms. Preferably, the microbial oil produced is enriched in the fatty acids of interest, meaning that it will contain at least about 20 % DHA or 10 % ARA by weight.

Any microorganism capable of producing a microbial oil containing DHA or ARA can be used in the present invention. These microorganisms can be identified by determining whether DHA or ARA oils are present in the fatty acid profiles of the harvested biomass from a culture of the microorganism. These profiles are typically obtained by gas chromatography of methyl ester derivatives of the fatty acids present in a sample.

As used herein, the term microorganism, or any specific type of microorganism, includes wild-type strains, mutant strains or recombinant strains. Wild-type and recombinant microorganisms designed to produce microbial cell oil containing DHA or ARA can be used to produce the DHA-containing and ARA-containing microbial oils. Such recombinant strains would include those designed to produce greater quantities of DHA or ARA in the single cell oil, greater quantities of total oil, or both, as compared to the quantities produced by the same wild-type microorganism, when provided with the same substrates. Microorganisms selected or designed to efficiently use more cost-effective substrates, while producing the same amount of single cell oil containing DHA or ARA as the wild-type microorganism, are particularly useful for preferred embodiments of the present invention.

For the production of DHA-containing microbial oils, species of photosynthetic algae, such as *Chattonella, Skeletonema, Thalassiosira, Isochrysis, Hymenomonas,* or *Cryptomonas* can be used. Preferred microorganisms are heterotrophic species of algae which include, but are not limited to, the *Dinophyceae,* for example, *Crypthecodinium*; or to fungi such as *Chytridiomycetes,* for example, *Thraustochytrium,* or *Schitzochytrium* or the *Oomycetes,* for example, *Mortierella, Saprolegnia* or *Mucor.*

Preferred microorganisms for producing DHA are dinoflagellates, including *Crypthecodinium.* Especially preferred is *Crypthecodinium cohnii,* an obligate heterotroph, which is described in U.S. Patent No. 5,407,957, issued April 18, 1995. *C. cohnii* is preferred because it produces fatty acids in which DHA is the only polyunsaturated fatty acid present in quantities greater than about 1 % of the total amount of polyunsaturated fatty acids, a quantity which is significant for carrying out the methods of the present invention. Samples of one strain of *C. cohnii,* which produces abundant levels of DHA, have been deposited with the American Type Culture Collection in Rockville, Maryland, and assigned ATCC accession number 40750.

Microorganisms useful for producing ARA include species of algae, such as *Porphyridium, Ochromonas* and *Euglene,* and fungi, such as *Thraustochytrium, Schizochytrium, Pythium* and *Mortierella.* Many of those species which make ARA also produce significant quantities of eicosapentaenoic acid (EPA) in addition. Unexpectedly, it has been found that *P. insidiosum* and *M. Alpina* produce ARA but are at least substantially free of EPA. "Substantially free" is defined to mean that the ratio of ARA to EPA is at least 5:1. Preferably, the ratio is at least 10:1. Most desirably, no more than 1% of the fatty acid content of the oil is EPA. As with fish oils, high EPA levels in dietary supplements result in a depression of the ability to form ARA from dietary linoleic acid. Furthermore, the administration of EPA-containing fish oils to patients, especially elderly, hypertensive or pregnant patients who may have increased prothrombin times, is undesirable because of the blood-thinning effects of EPA. Accordingly, while those fungal species producing both ARA and EPA can be utilized in the process of this invention, it is preferable to use species which do not produce significant quantities of EPA. Preferred species include *Pythium insidiosum* and *Mortierella alpina.* Both species are available commercially and strains are on deposit with the American Type Culture Collective in Rockville, Maryland, such as those having ATCC accession numbers 28251 and 42430, respectively.

Likewise, although microbial species producing both DHA and EPA can be utilized as a source of the DHA oil used in this invention, it is preferable to use species which are at least substantially free of EPA. Preferably, the ratio is at least 10:1. Most desirably, no more than 1 % of the fatty acid content of the oil is EPA.

### Production of DHA-Containing Oil

The DHA-producing microorganisms can be cultivated in a simple medium containing a carbon source, such as glucose, and a nitrogen source, such as yeast extract or peptone. Use of these components in a solution such as seawater provides economically significant growth rates and cell densities. During the course of a 3 to 5 day fermentation, for example, *C. cohnii* cell densities of at least 10 grams of biomass per liter of solution, and preferably from 20 to about 40 grams per liter, can be attained.

Although cultivation can occur in any suitable fermenter, preferably the organism is grown either in a stirred tank fermenter or in an air-lift fermenter. When a stirred tank fermenter is selected, agitation is provided using either Rushton-type high efficiency turbines or pitched-blade or marine impellers. Agitation and sparging renew the supply of oxygen to the microorganisms. The rate of agitation normally is increased as the biomass increases, due to the increased demand for oxygen. It is desirable to keep the tip speed at not grater than about 500 cm per sec, preferably not greater than about 300 cm per sec. Selection of strains of microorganisms which are capable of withstanding greater tip speeds without undergoing shear damage is within the purview of those of skill in the art. Control of foaming due to agitation is usually desirable, and suitable anti-foaming methods known to those skilled in the art are within the contemplation of this invention.

The organisms used for the production of DHA-containing oil can be grown in any suitable nutrient solution. As noted above, seawater is an acceptable medium for the nutrient solution for many organisms. The seawater can be either natural, filtered, or an artificial mix, each of which can be diluted with water to reduce salinities, such as 1/2 to 1/4 normal strength, or concentrated to 2 times normal strength. A preferred medium is Instant Ocean^{®} brand artificial seawater or, alternatively, a mixture of 4.5 to 20 g per liter NaCl, 1.23 g per liter MgSO₄7H₂O and 90 mg per liter CaCl₂ in water. Micronutrients can be added and may be required when using defined media. However, such micronutrients are known to those of skill in the art and generally are present in seawater or tap water. If the organism selected is heterotrophic, such as *Crypthecodinium* and *Thraustochytrium,* then a reduced carbon source is added. *Crypthecodinium* and *Thrmistocytrium* require a reduced carbon source for growth.

Preferably, after addition of the seawater medium to the fermentor, the fermenter containing the medium is sterilized and cooled prior to adding the nutrients and a seed culture of the microorganism to be cultivated. Although it is acceptable to sterilize the nutrients together with the seawater, sterilization in this manner can result in a loss of available glucose. The nutrients and microorganism can be added simultaneously or sequentially.

An effective seed concentration can be determined by those of skill in the art. When a stirred tank fermenter is used, the addition of a population of from about 0.05 to 1.0 grams of dry weight equivalent per liter at the beginning of the fermentation is preferred. For example, at least about 1 to 5 x 10⁶ cells of C. *cohnii* per ml would be suitable. Thus, for a 30-liter fermenter, 1 to 3 liters of seeding media, containing viable cells at a density of 10 to 20 grams dry weight per liter would be added.

Oxygen levels preferably are maintained at a dissolved oxygen of at least about 10% of air saturation level. Biosynthesis of DHA requires oxygen and, accordingly, higher yields of DHA require dissolved oxygen levels at from about 10 % to 50 % of air saturation levels. For example, agitation tip speeds of 150 to 200 cm per sec in combination with an aeration rate of 1 volume of air per volume of fermenter per minute (VVM) provides dissolved oxygen levels of from about 20 % to about 30 % at biomass densities of about 25 grams dry weight per liter of culture for *C. cohnii.* Higher cell densities may require higher dissolved oxygen levels, which can be attained by increased aeration rates by O₂ sparging or by increasing the air pressure in the fermenter.

Acceptable carbon sources are known to those of skill in the art. For example, carbon can be provided in the form of mono- or di-saccharides, such as sucrose, lactose, fructose, or glucose. Autotrophs utilize carbon dioxide as a carbon source. Many organisms also will grow on other reduced, more complex, carbon sources, such as molasses, high-fructose corn syrup, and hydrolyzed starch. Typically, a fermentation is initiated with about 20 to 50 grams per liter glucose. More glucose is added during the fermentation as required. Alternatively, from about 50 to 150 grams glucose per liter initially can be added, thereby minimizing the frequency of future additions. The amount of carbon source provided to other organisms can readily be determined by those of skill in the art.

In addition to a reduced carbon source, a nitrogen source, such as yeast extract or peptone, is provided to the medium. For example, Difco or Marco brand yeast extract and Sheftone brand peptone can be used. Yeast extract and peptone are organic nitrogen sources which also contain micronutrients. Other nitrogen sources easily can be determined by those of skill in the art. However, such compounds are generally more expensive than yeast extract. Any DHA- or ARA-producing algae strain variant capable of using urea, ammonia or nitrates as a nitrogen source can be used in this invention.

Typically, the fermentation is initiated with about 6 to 12 grams yeast extract per liter. More yeast extract can be added as required. A typical fermentation run requires from about 8 to 15 grams of yeast extract per liter over the course of the run. Accordingly, that amount of yeast extract can be added initially with a reduced need for further additions. The precise amount can be determined by those of skill in the art. Generally, the ratio of glucose to yeast extract is from about 2:1 to about 25:1.

Cultivation can be carried out at any life-sustaining temperature. Generally, microorganisms, such as *Crypthecodinium* or *Thraustochytrium* will grow at temperatures ranging from about 15°C to 34°C. Some fungi grow effectively at temperatures ranging from about 10°C to 80°C. Preferably, the temperature is maintained at about 20°C to 30°C. Strains which grow at higher temperatures are preferred, because they have a faster doubling time, thereby reducing total fermentation time. Appropriate temperature ranges for other microorganisms are readily determined by those of skill in the art.

Cultivation can be carried out over a broad pH range, typically from about pH 5.0 to 9.0. Preferably, a pH range of from about 6.0 to about 7.0 is used for the growth phase. A base, such as KOH or NaOH, is used to adjust the media pH prior to inoculation. During the later stages of the fermentation, the pH of the culture medium can increase or decrease as nutrients are utilized. If desired, the pH can be adjusted during the fermentation to correct alkalinity or acidity during the growth phase by adding an appropriate acid or base.

Production of the microbial cell oil is induced in the microorganisms by the induction of a stationary phase by allowing the culture to reach a phase of nitrogen depletion or phosphate depletion or by allowing the pH of the culture to rise. Yeast extract deficiencies can be caused by providing only a limited amount of yeast extract such that the medium is depleted of its nitrogen source, while available glucose levels remain adequate for growth. It is the caron source to nitrogen source ratio which promotes the efficient production of the single-cell oil. Using glucose and yeast extract as examples, a preferred ratio of carbon source to nitrogen source at the time of inoculation is about 10 to 15 parts glucose to 1 part yeast extract. Similar ratios for other carbon and nitrogen sources an be calculated by those of skill in the art.

After induction of oil production, the culture is grown from about 24 additional hours. During this period, the single-cell oil containing DHA is being synthesized and oil droplets are visible inside the cells when they are observed using a microscope. Those of skill in the art can readily calculate the time of fermentation required to achieve the expected amount of cell biomass based upon the added amount of yeast extract. When that time has passed, the culture is grown for an additional 24 hours and harvested. In general, for example, the *Crypthecodinium* or *Thraustochytrium* cells are cultivated for about 60 to about 90 hours, although this time is subject to variation.

Using the *Crypthecodinium* strain designated as ATCC accession number 40750, as an example, from about 15 to 30% of the resulting biomass comprises extractable oil. Strain selection can increase this percentage. Preferably, the oil comprises greater than about 70% triglycerides having, in general, the following fatty acid composition.
5-20% myristic acid (C_{14:0})
5-20% palmitic acid (C_{16:0})
5-15 % oleic acid (C_{18:1})
30-75 % DHA (C_{22:6})
The crude oil is characterized by a yellow-orange color and is liquid at room temperature. Desirably, the oil contains at least about 20 % DHA by weight, preferably about 40 % DHA by weight, and most preferably at least about 50 % DHA by weight.

The organisms are harvested by conventional means, known to those of skill in the art, such as centrifugation, flocculation, or filtration, and can be processed immediately or dried for future processing. In either event, the oil can be extracted readily with an effective amount of solvent. Suitable solvents can be determined by those of skill in the art. However, preferred solvents include pure hexane and supercritical fluids, such as supercritical CO₂. Certain lipophilic antioxidants, such as β-carotene, α-tocopherol, ascorbyl palmitate, and BHT can be added prior to extraction. These compounds help protect the oil from oxidation during the extraction and refining processes.

General extraction techniques using supercritical fluids have been developed for the extraction of oil from oil-rich plant seeds (McHugh, et al. , "Supercritical Fluid Extraction," Butterworth, 1986). However, these standard methods generally are not applicable to the extraction of microorganisms. For example, spray-dried algal cells have the consistency of flour, and he flow of supercritical CO₂ is restricted as the microorganism biomass is compressed. In addition, the cell walls of microalgae and fungi are chemically dissimilar to those of most seed oil material. In order to prevent the compression and allow efficient flow and extraction, the algal biomass can be mixed with from 0.1 to 5.0 parts of lipid-free structural agent, such as Celite, or diatomaceous earth. In a 50-ml reaction vessel at 450 Atm. and 100°C, 86% of the oil was extracted from *C. cohnii* in 25 minutes, and 100% was extracted in 85 minutes.

If the extraction solvent is hexane, a suitable ratio of hexane to dry biomass is about 4 liters of hexane per kilogram of dry biomass. The hexane preferably is mixed with the biomass in a stirred reaction vessel at a temperature of about 20° to 50°C for about 2 hours. After mixing, the biomass is centrifuged or filtered and separated from the hexane containing the oil. Alternatively, a wet biomass paste that is from 30% to 35% solids can be extracted directly with more polar solvents, such as ethanol, isopropanol, or mixtures of hexane and isopropanol.

The solvent is removed from the oil by distillation techniques known to those skilled in the art. Conventional seed oil processing equipment is suitable to perform the filtration, separation, and distillation. Additional processing steps, known to those of skill in the art, can be performed if required or desirable for a particular application. These steps also will be similar to those involved in conventional vegetable oil processing and will allow the separation of DHA-enriched polar lipid fractions.

### ARA-Containing Oil Production

ARA-producing fungi or algae are cultivated under suitable ARA-containing oil-producing cultivating conditions. If desired, the microorganism can be grown in a shake flask initially and then transferred to a fermenter. The composition of the growth medium can vary, but always contains carbon and nitrogen sources. A preferred carbon source is glucose, amounts of which can range from about 10 to 200 grams of glucose per liter of growth medium. Typically, about 50 grams per liter are utilized for shaker flask culture. The amount can be varied, depending upon the desired density of the final culture. Other carbon sources which can be used include molasses, high fructose corn syrup, hydrolyzed starch or any other low-cost conventional carbon source used in fermentation processes. Additionally, lactose can be provided as a carbon source. Thus, whey permeate, which is high in lactose and is a very low-cost carbon source, can be used as a substrate. Suitable amounts of these carbon sources can readily be determined by those skilled in the art. Usually, additional amounts of the carbon source need to be added during the course of the fermentation.

Nitrogen typically is provided in the form of yeast extract at a concentration of from about 2 to about 15 grams per liter of growth medium. Preferably, about 8 to 10 grams per liter are provided. Other nitrogen sources can be used, including peptone, tryptone, corn steep liquor, etc. The amount to be added of these sources can easily be determined by those skilled in the art. Nitrogen can be added throughout the cultivation or in a batch mode, *i.e.,* all at one time prior to cultivation.

After cultivation for 3 to 4 days at a suitable temperature, typically about 25°C to about 30°C, an amount of fungi or algae has grown which is sufficient for use as an inoculum in a conventional stirred tank fermenter or an air-lift fermenter. Fermentation can be carried out in batch, fed-batch, or continuous fermentation modes. The stirred tank fermenter is equipped with either a Rushton-type turbine impeller or, preferably, a marine-type axial impeller.

The fermenter is prepared by adding the desired carbon and nitrogen sources. For example, a 1.5 liter fermenter can be prepared by mixing about 50 grams of glucose and about 6 grams of yeast extract per liter of water. As previously discussed, other carbon or nitrogen sources or mixtures thereof can be used.

The reactor containing the nutrient solution should be sterilized by, for example, heating prior to inoculation, as described above in the discussion of microorganism cultivation for the production of DHA. After cooling to about 30°C, the inoculum can be added and cultivation initiated. Gas exchange is provided by air-sparging. The air-sparging rate can vary, but preferably is adjusted to from about 0.5 to about 2.0 volumes of air per volume of fermenter per minute. Preferably, the dissolved oxygen level is kept at from about 10% to about 50% of the air saturation value of the solution. Accordingly, adjustments in the sparging rate may be required during cultivation.

Agitation is desirable during fermentation. The agitation is provided by the impeller. Agitation tip speed preferably is set within the range of from about 50 cm per sec to about 500 cm per sec, preferably from about 100 to 200 cm per sec.

In general, the amount of inoculum used in a fermentation can vary. Typically, a logarithmically growing culture that is from about 2% to about 10% of the total volume of the medium in the fermenter can be used as an inoculum.

Nutrient levels should be monitored. When glucose levels drop below 5 grams per 1, additional glucose should be added. A typical cultivation cycle utilizes about 100 grams of glucose and about 15 grams of yeast extract per liter. It is desirable to deplete the nitrogen during the course of the cultivation, as this enhances oil production by the fungi or algae. This is especially true when *M. alpina* is used as the production organism.

Occasionally, the culture will produce an excessive quantity of foam. Optionally, an antifoaming agent, such as agents known to those skilled in the art, for example, Mazu 310®, can be added to prevent foaming.

The temperature of cultivation can vary. However, those microorganisms which produce both ARA and EPA tend to produce less EPA and more ARA when cultivated at higher temperatures. For example, when *Mortierella alpina* is cultivated at less than 18°C, it begins to produce EPA. Thus, it is preferable to maintain the temperature at a level which induces the preferential production of ARA. Suitable temperatures are typically from about 25°C to about 30°C.

Preferably, cultivation continues until a desired biomass density is achieved. A desirable biomass is about 15-40 grams per liter of the organism. Such a biomass typically is attained within 48 to 72 hours after inoculation. At this time, the organisms typically contain about 5% to 40 % complex lipids, of which about 10% to 50% is ARA, and can be harvested.

Harvesting can be done by any suitable method, such as filtration, centrifugation, or flocculation. Because of lower cost, filtration may be preferred.

After harvesting, the biomass can be extracted without drying. Optionally, the biomass can have any residual water removed, as by vacuum drying, fluid-bed drying, or lyophilization, prior to extraction. If the water is removed, it is preferable to use nonpolar solvents to extract the ARA-containing oil. While any nonpolar extract is suitable, hexane is preferred. Supercritical fluids, such as CO₂ or NO, as discussed above, also can be used for extraction of ARA-enriched oils from algae and fungi. Although fungi such as *M. alpina* are unexpectedly difficult to extract with CO₂, as much as 89 % of the oil of a fungal biomass can be recovered at temperatures about 90°C and pressures of 400 Atm. Alternatively, the wet biomass, which typically contains about 30 to 50 % solids, can be crumbled and extracted directly using polar solvents, such as ethanol, isopropyl alcohol, or a mixture of hexane and isopropyl alcohol.

A preferred method of aqueous extraction involves mixing the biomass with the polar solvent isopropyl alcohol in a suitable reaction kettle. Such kettles are known. The use of three to six parts of solvent per part of biomass is desired. Most preferably, the mixing is done under nitrogen or with the addition of antioxidants, such as β-carotene, α-tocopherol, ascorbyl palmitate, or BHT to prevent the oxidation of the ARA in the lipid extract.

The solvent is removed from the oil, as discussed in the section above regarding the production of a DHA-containing oil. Additional steps to further purify the oil also can be performed. Yields can vary from about 5-50 grams of ARA-containing oil per 100 grams of dried biomass. In the case of *M. alpina,* 10 to 50 grams of triglyceride per 100 grams of dry biomass can be obtained. In the case of *Ochromonas,* 5 to 20 grams of triglyceride per 100 grams of biomass can be obtained.

Preferably, the oil from *M. alpina* comprises greater than about 70 % triglycerides having, in general, the following fatty acid composition:
5 - 15 % palmitic acid
15-20% stearic acid
5-10% oleic acid
6-10% linoleic acid
2-10% linolenic acid
2-10% dihomo-gamma linolenic acid
40-50% arachidonic acid

### Administration of DHA-and ARA-Containing Oils

In accordance with this invention, DHA-containing microbial oils, ARA-containing microbial oils, or suitable combinations of these oils, are administered to patients affected by a neurological disorder or other disease characterized by depressed levels of DHA and/or ARA in the blood or tissues in comparison to the levels found in healthy individuals. For example, depression of DHA levels in cardiac or neuronal tissues, particularly in the cells having excitable membranes, can lead to pathology. The method of this invention includes administration these oils to patients affected by neurological, cardiac, or liver disorders where depressed levels of DHA and/or ARA are characteristic of the disease. The therapeutic compounds of this invention are also useful for improving the condition of patients suffering as a result of inborn errors of metabolism which result, among other things, in depression of DHA and/or ARA levels in one or more tissues. Such diseases include multiple sclerosis, cerebral palsy, amyotrophic lateral sclerosis, phenylketonuria and cystic fibrosis. It is not contemplated that the method of this invention will provide a complete cure for each and every disease listed, but increasing the level of the HUFA which is depressed will be of at least palliative benefit.

The oils are administered in a manner that results in increasing the level of DHA and/or ARA in the tissues where depressed levels of these HUFAs contributes to the pathology of the disorder (hereinafter the "target" tissues). Appropriate target tissues may be identified based on the abnormal or pathologic functional characteristics exhibited by the tissues in conjunction with depressed levels of DHA and/or ARA. Deviations from normal will be readily recognized by the skilled clinician familiar with the pathologic characteristics of particular diseases.

While treatment is preferably monitored for dose adjustment by following the level of DHA and/or ARA in the target tissue, the skilled clinician may alternatively monitor the level of HUFAs in a surrogate tissue, such as blood. Blood is a preferred surrogate tissue because it is relatively easy to obtain samples throughout treatment and monitor HUFA levels. HUFA levels can also be monitored in fractions of whole blood, such as serum, plasma, erythrocytes, etc. The specific course of treatment administered can be determined based on normalization of serum and erythrocyte DHA and ARA levels. These serum levels of DHA and ARA are thought to reflect the long-chain polyunsaturated fatty-acid compositions of neurological membranes. As will be apparent to the skilled clinician, if the level of the desired HUFA in the surrogate tissue is normal, then that level will be raised above normal by treatment which increases the level of HUFA in the target tissue. In some cases, serum levels of ARA and DHA may need to be increased to 4 to 5 times the levels which are considered to be normal in the general population in order to see a therapeutic effect. Patients suffering from disorders involving such conditions as retinitis pigmentosum or senile dementia may respond to the administration of DHA-containing oil alone, while patients suffering from such conditions as adrenoleukodystrophy, diabetes-induced neuropathy, or schizophrenia may respond more favorably to the administration of a combination of a DHA-containing oil and an ARA-containing oil. Still other patients may benefit from the administration of an ARA-containing microbial oil alone.

The course of treatment can be followed by measuring levels of the fatty acid(s) of interest in the serum of treated patients. For some patients, it will be possible to follow the normalization of DHA or ARA levels in neural tissue by measuring the levels of DHA and ARA in erythrocytes or in serum lipids during treatment.

### Therapeutic Mixtures to Restore Lipid Balance

As provided by this invention, therapeutic oil mixtures may be formulated to correct imbalances in the fatty acid composition of one or more tissues of a patient, in particular imbalances in the relative amounts of highly unsaturated fatty acids (HUFA: fatty acids having 20 or 22 carbons and at least three double bonds). The method of correcting imbalances requires administering a mixture of triglycerides, the mixture containing esterified fatty acids in the desired ratio. Preparation of such mixtures has only recently become possible, with the development of microbial fermentations, such as those described above, which produce triglyceride oils containing predominantly one HUFA with little or none of other HUFAs among the fatty acid residues in triglyceride form. An oil which has essentially no HUFA other than DHA, for instance, can be blended with fish oil to give a mixture that is disproportionately composed of DHA residues (i.e., highly enriched in DHA residues), and administration of this oil will result in increased DHA in the patient's tissues. Because blends of triglyceride oils with tailored ratios of HUFAs could not be obtained prior to the development of the microbial specialty oils described herein, the benefits of providing such tailored mixtures to patients have not heretofore been recognized.

One embodiment of the present invention provides a method for restoring normal fatty acid profile with respect to HUFA content in tissue of a patient exhibiting a lipid imbalance. For example, when the tissue exhibiting the lipid imbalance is blood, the fatty acid profile of the patient's serum may be determined before initiation of therapy (the pre-therapy fatty acid profile), and the patient's pre-therapy fatty acid profile compared to the fatty acid profile for normal individuals (i.e., individuals not overtly suffering from a disease characterized by fatty acid imbalance). The "normal" fatty acid profile will usually be determined by each clinical analysis facility, as a range based on historical data from their local patient population. A therapeutic mixture of triglycerides is prepared in which the HUFAs which are below normal in the patient's profile are provided at high levels.

The therapeutic mixture may be prepared by computing a "deviation" for each HLTFA in the patient's pre-therapy serum lipid profile, where the deviation is the difference between level of each HUFA in normal serum and level of each HUFA in the patient's serum. The therapeutic supplement can then be prepared by combining a number of individual HUFA-containing oils in relative amounts that result in the amount of each HUFA in the therapeutic supplement being proportional to the deviation of that HUFA, as calculated previously. Preferably, at least one individual HUFA-containing oil included in the mixture will be substantially free of all HUFAs except one; the availability of microbial oils which contain essentially only one HUFA provides the flexibility to formulate such therapeutic mixtures for individual syndromes and even individual patients.

The therapeutic mixture is then administered to the patient in an amount which is effective to restore normal serum lipid profile to the patient, such that the level of each HUFA in serum is not elevated above the normal serum level. This type of therapy cannot be achieved using only natural extracts that have multiple HUFAs present, because administering enough of the therapeutic mixture to raise the relative amount of one HUFA in the patient's serum will inevitably raise the amount of the other HUFAs present in the oil above the desired level. However, by including one or more microbial oils in the therapeutic mixture, a supplement can be prepared containing, e.g., fish oil to supply relatively small amounts of most of the desired HUFAs with a higher excess of a particular HUFA supplied by the microbial oil. The mixed oil can be administered by injection, intravenously, intraperitoneally, etc., or by mouth as capsules, as a liquid or in an oil-containing food, in a nasal spray or transdermally.

Therapy using tailored mixtures of HUFA-containing triglycerides is superior to administration of fish oil because increasing the absolute amount of a particular fatty acid taken up by the patient using fish oil necessitates increasing the amount administered of all the other fatty acids present in the oil. In contrast, the mixtures of this invention can be tailored by blending oils which are high in particular fatty acids and low in others to provide only one particular HUFA in excess amounts, keeping the amounts of the other HUFAs at the level which was present in the diet before initiation of therapy. This is important because individual HUFAs are processed via different pathways, and result in, for example, increased levels of different prostaglandins. Thus, excess ARA can induce platelet aggregation (through prostaglandin PGE₂ via the cyclooxygenase pathway) while excess EPA tends to reduce platelet aggregation. Omega-3 HUFA generally tend to have opposite effects to ω-6 HUFA. Selective increases in the desired HUFA without increasing other HUFAs, as can be accomplished by the tailored mixtures of this invention, will enhance the therapeutic efficacy. On the other hand, increasing the amount of fish oil administered to the patient will tend to increase the serum level of all HUFAs.

While the preparation and use of therapeutic mixtures has been discussed in relationship to correcting the lipid balance of serum, such mixtures can also be used to correct lipid imbalance in patient tissues other than blood. For example, disorders involving cells in the heart muscle (cardiac myocytes), where the DHA level is too low in excitable membranes of these cells, may be treated by administering therapeutic compositions containing triglycerides high in DHA to increase the DHA content of the excitable membranes, preferably to the level found in normal heart muscle. Such therapeutic compositions may contain DHA as the only HUFA, or may be made up of a high HUFA source, such as fish oil blended with a microbial triglyceride oil that is high in DHA and essentially free of EPA (i.e., the DHA level is at least 10 times the EPA level, preferably 20 times the EPA level). A therapeutic composition essentially free of EPA is particularly preferred if, in order to increase the level a particular HUFA in the target tissue, it is necessary to administer enough of the therapeutic composition to raise the level of the HUFA in serum substantially above normal.

Although the DHA- and/or ARA-containing oils can be administered to patients directly, more commonly, they will be combined with one or more pharmaceutically acceptable carriers and, optionally, other therapeutic ingredients. Acceptable carriers are those which are compatible with the other components of the formulation and not deleterious to the patient.

Formulations include those suitable for oral, nasal, topical, or parenteral (including subcutaneous, intramuscular, intravenous, and intradermal) administration. It will be appreciate that the preferred formulation can vary with the condition and age of the patient. The formulations conveniently can be presented in unit dosage form, e.g., emulsions, tablets, and sustained release capsules, and can be prepared by any suitable pharmaceutical method.

Formulations of the present invention suitable for oral administration can be presented as discrete units, such as capsules or tablets, each of which contains a predetermined amount of DHA or ARA oil or a predetermined amount of a suitable combination of DHA and ARA oils. These oral formulations also can comprise a solution or a suspension in an aqueous liquid or a non-aqueous liquid. The solution can be an emulsion, such as an oil-in-water liquid emulsion or a water-in-oil liquid emulsion. The oils can be administered by adding the purified and sterilized liquids to a prepared enteral formula which is then placed int he feeding tube of a patient who is unable to swallow.

In one preferred embodiment, the DHA or ARA microbial oil is incorporated into gel capsules, such as those described in Example 6. However, it will be recognized that any known means of producing gel capsules can be used in accordance with the present invention.

Compressed tablets can be prepared by, for example, mixing the microbial oil(s) with dry inert ingredients such as carboxymethyl cellulose and compressing or molding in a suitable machine. The tablets optionally can be coated or scored and can be formulated so as to provide slow or controlled release of the active ingredients therein.

Other formulations suitable for topical administration include lozenges comprising DHA oil, ARA oil, or a combination thereof in a flavored base, usually sucrose and acacia or tragacanth.

Formulations suitable for topical administration to the skin can be presented as ointments, creams and gels comprising the DHA and/or ARA oil(s) in a pharmaceutically acceptable carrier. A preferred topical delivery system is a transdermal patch containing the oil to be administered.

In formulations suitable for nasal administration, the carrier is a liquid, such as those used in a conventional nasal spray or nasal drops.

Formulations suitable for parenteral administration include aqueous and non-aqueous sterile injection solutions which optionally can contain antioxidants, buffers, bacteriostats and solutes which render the formulation isotonic with the blood of the intended recipient; and aqueous and non-aqueous sterile suspensions which can include suspending agents and thickening agents. The formulations can be presented in unit-dose or multi-dose containers. A preferred embodiment of the present invention includes incorporation of the DHA and/or ARA oil(s) into a formulation for providing parenteral nutrition to a patient.

The microbial oil compositions of the present invention need not be administered as a pharmaceutical composition. They also can be formulated as a dietary supplement, such as a vitamin capsule or as food replacement in the normal diet. The microbial oils can be administered as a cooking oil replacement formulated so that in normal usage the patient would receive amounts of DHA and/or ARA sufficient to elevate the concentrations of these fatty acids in the serum and in membranes of affected neural tissues to normal or near-normal levels. A special emulsion type margarine could also be formulated to replace butter or ordinary margarine in the diet. The single cell microbial oils could be added to processed foods to provide an improved source of ω-3 and ω-6 unsaturated fatty acids. The oil can be microencapsulated using gelatin, casein, or other suitable proteins using methods known in the art, thereby providing a dry ingredient form of the oil for food processing. Such methods of administration can be preferred in the case of a person known to have a genetic predisposition to a disorder associated with a DHA or ARA metabolic deficiency such as a neurodegenerative disease, for example Huntington's disease or Alzheimer's disease. Such methods of administration are also preferred in prophylactic therapy for patients at risk for atherosclerosis, and generally for therapy aimed at correcting lipid imbalance in serum or in other target tissues. Providing such an individual with a dietary replacement can provide a significant prophylactic effect, delaying the onset of symptoms of a particular disorder. The administration of the long chain polyunsaturated fatty acids DHA and ARA offer a significant advantage over merely obtaining linoleic and linolenic acid, the precursors of these fatty acids, from standard foods or specialty oils such as primrose or borage oil. The administered DHA and ARA are already present in their active forms so that the patient is not required to metabolize dietary precursors. This results in the effective doses which are significantly lower than those of the precursors which would be required to produce the therapeutic effect.

It should be understood that in addition to the ingredients particularly mentioned above, the formulations of this invention can include other suitable agents such as flavoring agents, preservatives and antioxidants. In particular, it is desirable to mix the microbial oils with an antioxidant to prevent oxidation of the DHA or ARA. Such antioxidants would be food acceptable and could include vitamin E, carotene, BHT or other antioxidants known to those of skill in the art.

The daily dose of the compositions of the present invention to be provided to a patient will depend upon the extent of the DHA and/or ARA deficit identified by serum lipid analysis prior to the introduction of the therapy. Typically, the initial dose provided to a patient of greater than 50 pounds will be in the range of about 50 mg DHA to 5000 mg DHA per day. A preferred maintenance dose is about 500 mg DHA per day. For example, if the DHA oil to be used is 50% enriched in DHA, such a dose would correspond to the addition of about 1000 mg of oil per day.

The daily dose of ARA provided to the patient of greater than 50 pounds will be 50 mg ARA to 5000 mg per day. A preferred maintenance dose would be 500-1000 mg per day. If the ARA oil to be used is 50% enriched in ARA, such a dose would correspond to the addition of about 1000-2000 mg of ARA oil per day. Doses of a suitable combination of the DHA and ARA containing oils will be 1000 mg of DHA and 1000 mg of ARA per day.

Desirably, the patient's serum fatty acid profiles are reviewed after about four weeks of this daily therapy. Subsequent doses then can be modified in response to the observed level of plasma lipid or red blood cell DHA and ARA and in response to observed clinical responses to the therapy. Patients with peroxisomal disorders can have red blood cell levels of DHA of only 1-3 µg DHA per ml. of plasma. Normal target values range from about 10 to 30 µg of DHA per ml of plasma. Normal target values of circulating ARA range from about 75 to about 120 µg ARA per ml. of plasma. Once normalized level(s) of the circulating fatty acid(s) of interest have been achieved, the daily dose of oil(s) can be modified to maintain the circulating DHA and/or ARA at a desirable level.

As noted above, in order to treat certain neurological disorders, it may be desirable to raise the level of circulating DHA and/or ARA in the blood to 4 to 5 times normal levels. The levels of circulating DHA and ARA, therefore, can be raised to about 120-150 µg/ml and about 480-600 µg/ml, respectively.

Although not wanting to be bound by any specific theory, it is the inventor's belief that the administration of DHA is effective for treating neurological disorders because of its ability to regulate calcium uptake by neuronal cells. A depolarization of the neuronal cell results in elevated levels of intracellular calcium, causing the activation of a phospholipase and resulting in the release of free DHA from the cell membrane. This free DHA acts as a calcium channel blocker, thereby limiting calcium entry into the cell. Thus, the level of DHA present in the neuronal cell membrane, and thereby available for activation-induced release of these long chain polyunsaturated fatty acids, may control intracellular calcium levels. If a deficiency of DHA exists, intracellular calcium levels rise, and the production of amyloid plaque protein may be stimulated. Furthermore, high intracellular calcium stimulates the phosphorylation of the microtubule associated tauprotein, resulting in the development of neurofibrillar tangles.

### Alzheimer's Disease

A marked decrease in DHA levels in the brain has been reported in Alzheimer's disease. The loss of DHA in Alzheimer's disease may be casually related to the development of dementia, and recent studies have suggested that DHA may stimulate synaptic plasticity mediated via NMDA glutamate receptors (Nishikawa, et al. (1994), J. Physiol., 475:83-93). Increase of DHA levels in neuronal tissue of elderly patients, preferably by supplementation with a diet high in DHA, could alleviate the biochemical defect (decrease of DHA content) as well as the cognitive defect which results in Alzheimer's disease.

The present invention provides a method for achieving such an increase in DHA levels, by administering a therapeutic composition (usually a dietary supplement) containing triglycerides highly enriched in DHA. Using the compositions provided in this invention, DHA level in neuronal tissue can be increased without the detrimental effects of excess EPA described above, which would result from attempting to increase DHA by feeding fish oil.

Serum lipids are the most probable source of the DHA and ARA incorporated into neuronal cells, since serum lipids act as the transport or carrier system for fatty acids in general. Studies in animals and in humans have shown that high levels of DHA and ARA in the serum are correlated with high levels of DHA and ARA in the brain. Therefore, elevating the DHA and ARA concentrations in the composition of total serum lipids, by providing supplemental dietary microbial oils enriched in these components, should increase the delivery of DHA and ARA to target neuronal tissues.

The role of ARA in neuronal function is less clear, although it too is a major component of neurological membranes. Many neurological disorders exhibit a deficiency of both DHA and ARA. The object of this invention is to supplement levels of both these components, using DHA and ARA from microbial oil to normalize both of these important fatty acids. The supplementation of DHA and ARA without any significant quantities of EPA is an important aspect of this invention, as the EPA levels in neurological tissues generally are low and supplementation with EPA will depress ARA levels, and may be contraindicated in certain instances. Therapeutic administration of the DHA oil in combination with the ARA oil may be beneficial in maintaining or establishing a ratio of ω-3 to ω-6 long chain polyunsaturated fatty acid in the body comparable to that in normal healthy individuals. Lowering of Serum Triglycerides

Various angiographic and epidemiologic studies have shown that plasma triglyceride concentration is a factor in predicting atherosclerotic risk (Assmann, et al., 1992, "Relation of High-Density Lipoprotein Cholesterol and Triglycerides to Incidence of Atherosclerotic Coronary Artery Disease (the PROCAM Experience)," Am. J. Cardiol., 70:733-737; Drexel, et al., 1994, "Plasma Triglycerides and Three Lipoprotein Cholesterol Fractions are Independent Predictors of the Extent of Coronary Atherosclerosis," Circulation, 90:2230-2235; Sharrett, et al., 1994, "Associations of Lipoprotein Cholesterols, Apolipoproteins A-I and B, and Triglycerides with Carotid Atherosclerosis and Coronary Heart Disease. The Atherosclerosis Risk in Communities (ARIC) Study," Arterioscler. Thromb., 14:1098-1104; Tenkanen, et al., 1994, "The Triglyceride Issue Revisited. Finding from the Helsinki Heart Study," Arch. Intern. Med., 154:2714-2720). In particular, cardiovascular risk is substantially increased among persons having elevated triglycerides accompanied by relatively low levels of cholesterol in high density lipoproteins (HDL-C; see Assmann, et al., 1992; Tenkanen, et al., 1994). Such lipid abnormalities may be accentuated during consumption of a low-fat, high carbohydrate diet, the diet recommended by the National Cholesterol Education Program for persons with elevated LDL-C (Mensink, et al., 1992, "Effect of Dietary Fatty Acids on Serum Lipids and Lipoproteins. A Meta-Analysis of 27 Trials," Aneriscler. Thromb., 12:399-406).

Administration of supplements containing EPA and DHA to patients with elevated triglyceride concentrations have consistently shown that such supplements have a hypotriglyceridemic effect. However, the effects on total cholesterol, LDL-C and HDL-C have been variable in studies with such supplements. Reasons offered for the inconsistent findings include variations in the cholesterol and saturated fat content of the supplements used, and the lipoprotein phenotype of the patients studied (Davidson, et al., 1991, "Marine Oil Capsule Therapy for Treatment of Hyperlipidemia," Am. J. Clin. Nutr., 51:399-406), but important questions regarding the minimal effective dose and the relative importance of EPA vs. DHA in the hypolipidemic actions of these supplements were not satisfactorily addressed. Additionally, the doses used in some of these studies (up to 30 grams per day) were impractical for use clinical settings because they are associated with side effects and weight gain.

In a cholesterol-fed rat model, EPA lowered triglyceride levels, but increased LDL-cholesterol (LDL-C), while DHA significantly lowered LDL-C, not triglyceride levels (Yoshiki, et al., 1984, "Differential Effects of Dietary Eicosapentaenoic and Docosahexaenoic Fatty Acids on Lowering of Triglyceride and Cholesterol Levels in the Serum of Rats on Hypercholesterolemic Diet, "J. Nutr. Sci. Vitamin,30:357-372). The present inventor has interpreted this data to indicate that EPA may be primarily responsible for the rise in LDL-C demonstrated in the human clinical trials. In human subjects, purified DHA has been shown to result in a greater reduction of platelet aggregation than EPA ingestion (Schacky, et al., 1985, "Metabolism and Effects on Platelet Function of the Purified Eicosapentaenoic and Docosahexaenoic Acids in Humans, "J. Clin. Invest. , 76:2446-2450). In general, however, the beneficial aspects associated with marine oil are still observed for supplements containing DHA with little or no EPA. Therefore the combination of DHA with EPA in fish oil is of little additional therapeutic benefit or may even negate the positive attributes of DHA, while supplements containing DHA with little or no EPA provide a clinically useful adjunct to standard dietary therapy by favorably altering triglyceride and HDL-C concentrations. Additionally, DHA-enriched supplements may limit or reverse the increase in triglyceride concentration seen when bile acid sequestrants are administered to persons with Type IIb dyslipidemia.

### Stabilization of Cardiac Arrhythmia

Hallaq and Leaf reported that fish oil fatty acids EPA and DHA, but not ARA, can prevent contraction and arrhythmia induced by toxic levels of ouabain in rat heart myocytes. They determined that EPA and DHA modulated the dihydropyridine-sensitive calcium channel (Pepe, et al. (1994), Proc. Natl. Acad. Sci. USA, 91:8832-8836). When the myocytes were treated with agonists of the channel, supplementation of the medium with EPA and DHA reduced calcium influx, and lowered cytosolic free calcium concentration, as well as preventing contracture. When the myocytes were treated with antagonists of the dihydropyridine calcium channel which inhibit spontaneous contractions, EPA and DHA prevented the inhibitory effect. This data further supports the role of DHA in normalizing calcium metabolism by excitable membranes, as discussed above for neuronal cells.

Men told to eat fish several times per week after a first heart attack suffer fewer subsequent fatal heart attacks than those who do not eat fish. McLennan and Charnock demonstrated that fish oil feedings essentially prevent ventricular fibrillation associated with temporary experimental occlusion of a coronary artery in rats and monkeys (see Charnock, et al. (1992), in Sinclair, et al., eds., "Essential Fatty Acids and Eicosanoids: Invited Papers from the Third International Congress," Am. Oil Cem. Soc., p. 239; see also Billman, et al. (1994), Proc. Natl. Acad. Sci. USA, 91:4427-4430). In conjunction with these demonstrations, the improved post-heart attack prognosis for men who eat fish may be assumed to be due to reduction in episodes of ventricular fibrillation correlated with fish oil consumption. Thus, the therapeutic compositions containing DHA according to this invention may also be administered prophylactically to post-heart attack patients, with reduction in side effects from EPA co-supplementation that would occur were fish oil to be administered.

The present invention having been generally described, reference is had to the following non-limiting specific examples.

### Example 1

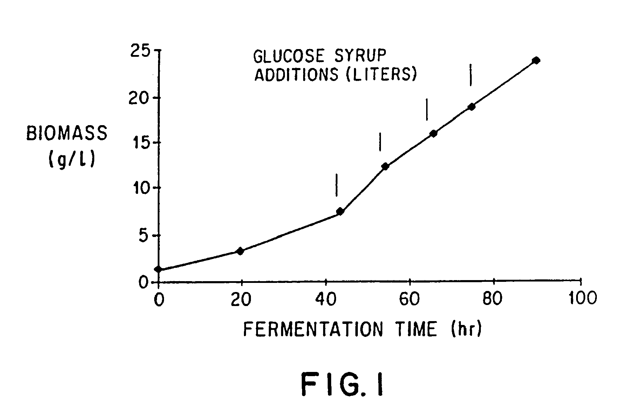

A medium of one half strength artificial seawater made by combining 4.3 kg of Instant Ocean^{®} with 230 liters of tap water was loaded into a 350 liter stirred tank fermentor. The fermentor containing the medium was sterilized and cooled to 28°C. 6.8 liters of concentrated yeast extract at a concentration of 400 grams per liter, 12.5 liters of glucose syrup at a concentration of 400 grams per liter and 30 liters of *C. cohnii* inoculum from a seed fermentor at a concentration of 10⁶ cells per ml or a biomass density of about 1.3 grams per liter were added to the medium. Agitation was set at a tip speed of 73 cm per sec and aeration was set at 1 VVM, which is equivalent to 280 liters per minute. An additional 12 liters of glucose syrup was added after about 44 hours and another 43 liters was added over the next 32 hours. Thus, 67.5 liters of glucose syrup were added in total. The glucose additions and the cell growth are depicted graphically in Figure 1.

To maintain the dissolved oxygen at greater than 20%, the agitation tip speed was increased to 175 cm per second at 44 hours and to 225 cm per sec at 55 hours. At 76 hours, the tip speed was decreased to 150 cm per second. The culture was grown for an additional time sufficient to convert the final charge of glucose into cellular oil, then harvested. The harvested cells were dried to about a 4 % moisture content. Hexane was added to the dried biomass and stirred in a glass kettle for 2 hours at 25°C. A rotary evaporator was used to remove the hexane, producing about 700 grams of crude DHA-containing oil.

### Example 2

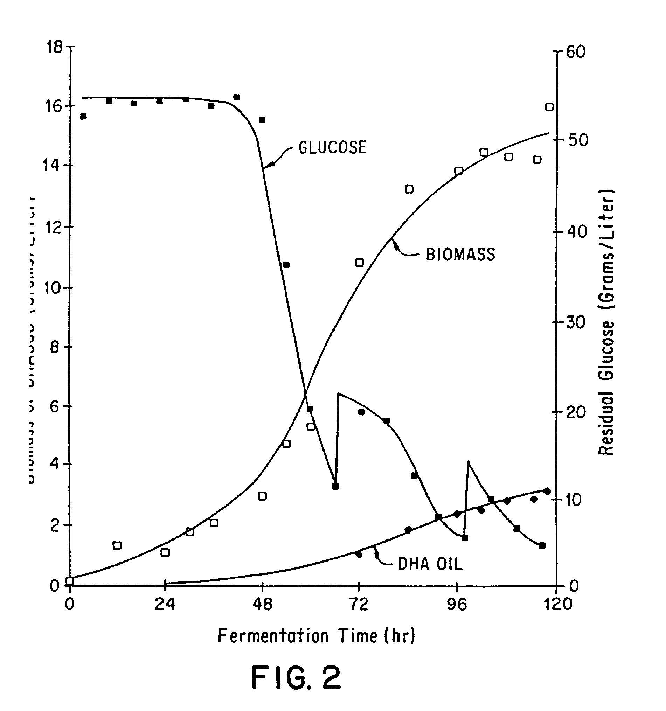

Sixty kg of yeast extract, 45 kg of NaCl, 12.3 kg of MgSO₄·7H₂O, and 0.9 kg of CaCl₂·2H₂O in 7,000 liters of water were loaded into a 15,000 liter fermentor. After this solution was sterilized, 3,000 liters of a sterilized glucose solution at a concentration of 650 kg of glucose per 3,000 liters of volume was added. The initial pH of the medium was 6.3, the temperature was 28°C, aeration was 0.5 to 1.0 VVM, the vessel back pressure was set to 0.2 bar, and the agitation tip speed was set to 120 cm per seconds before the vessel was inoculated with 300 liter of an inoculum culture of *C. cohnii* which had attained a cell density of about 60 x 10⁶ cells per ml, which is equivalent to 4 to 5 grams of dry weight of biomass per liter of culture in the inoculum tank. During the course of the fermentation, a food grade antifoam, such as Dow 1520 was added as needed and the pH was held at 6.3 using either 8 N H₂SO₄ or 4 N NaOH as needed. The dissolved oxygen level was maintained at greater than 20% of air saturation by increasing the vessel back pressure and agitation. Additional glucose additions were required at 93 hours and at 111 hours to maintain the glucose levels above 5 grams per liter. The course of the fermentation is shown in Figure 2, At 119 hours, the fermentor was cooled to 17°C and the fermentation broth was processed through a centrifuge. A 608 kg slurry containing 25 % solids was recovered. The slurry was spray dried, producing about 150 kg dry algal powder which contained about 30 to 40 kg of oil with a DHA content of 40 to 45 %.

The dry algal powder was extracted with hexane using standard vegetable oil extraction equipment and methods. Following the removal of the solvent, the crude oil was degummed by the adding water at 50°C. The degummed oil was collected by centrifugation and refined by mixing with caustic base and phosphoric acid at 55°C for one hour. The refined and degummed oil was then collected by centrifugation and gently bleached at 90°C by the addition of citric acid and bleaching clay. Filtration of the bleaching clay produced the refined, bleached oil (RB-oil) with a peroxide value of less than 5 mEq per kg. The RB-oil was deodorized by high vacuum short path distillation and the resulting deodorized RB-oil (RBD-oil) was then ready for encapsulation, tableting, or bulk shipping. The resulting oil had a peroxide value less than 1 mEq per kg, a free fatty acid content of less than 0.05%, a DHA content of 45 to 47%, and an iodine number of about 200.

### Example 3 Preparation of Thraustochytrium aurum lipid

2.5 grams of NaCl, 5 grams of MgSO₄·7H₂O, 1 gram of KCI, 0.1 grams of KH₂PO₄, 0.2 grams of CaCO₃, 0.2 grams of (NH₄)₂SO₄, 2.0 grams of sodium glutamate in 1 liter of water were loaded into a 1.7 liter stirred tank fermentor. After the tank was sterilized, a sterile solution containing 10 µgrams of thiamine-HCl, 0.1 grams of NaHCO₃, and 10 µgrams of vitamin B₁₂ was addedthiamine B₁₂ followed by the addition of 150 ml of sterile 30% glucose and 50 ml of sterile 10 % yeast extract. The pH was adjusted to 6.0, the sparging was adjusted to 1.0 VVM, and agitation was adjusted to 300 rpm before inoculation with 100 ml of a 5-day old shake flask culture of *Thraustochytrium aurum* grown in the same medium. The culture was harvested after 9 days to yield about 4 grams dry weight of biomass. The DHA content of the lipid in the biomass is 10 to 15%.

### Example 4 Preparation of Pythium insidiosum lipid

In an 80 liter (gross volume) fermentor, 51 liters of tap water, 1.2 kg glucose, 240 grams of yeast extract and 15 ml of MAZU 210S® antifoam were combined. The fermentor was sterilized at 121°C for 45 minutes. An additional 5 liters of condensate water were added during the sterilization process. The pH was adjusted to 6.2, and approximately 1 liter of inoculum at a cell density of 5 to 10 grams per liter of *Pythium insidiosum* (ATCC #28251) then was added. The agitation rate was adjusted to 125 RPM corresponding to a tip speed of 250 cm per second and the aeration rate was set at 1 SCFM (standard cubic feet per minute). At hour 24 in the operation the aeration rate was increased to 3 SCFM. At hour 28 an additional 2 liters of a 50% by weight glucose syrup were added. At hour 50 the fermentor was harvested, resulting in a yield of about 2.2 kg wet weight of biomass, which was approximately 15 grams of dry weight per liter of culture. The harvested biomass was squeezed to a high solids cake, comprising approximately 50 % solids, on a suction filter before it was freeze dried. The dried biomass was ground with a mortar and pestle and extracted with 1 liter of hexane per 200 grams of dry biomass at room temperature under continuous stirring for 2 hours. The mixture then was filtered and the filtrate evaporated, yielding about 5 to 6 grams of crude oil per 100 grams of dry biomass. The biomass was reextracted with 1 liter of ethanol per 20 grams of dry biomass for 1 hour at room temperature, filtered, and the solvent evaporated, yielding an additional 22 grams of crude oil per 100 grams of dry biomass. The second fraction was predominantly phospholipids whereas the first fraction contained a mixture of phospholipids and triglycerides. The combined fractions produced an oil containing about 30 to 35 % arachidonic acid and no detectable EPA.

### Example 5 Preparation of Mortierella alpina lipid

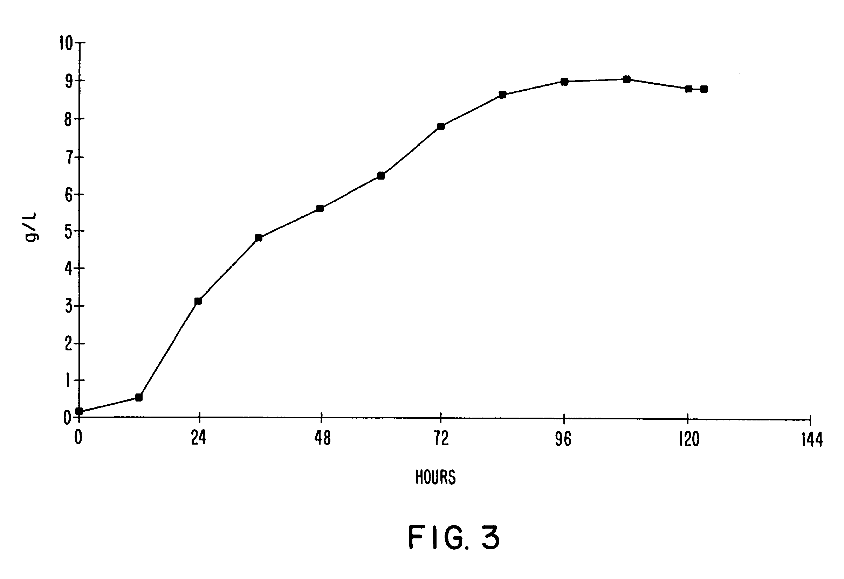

A 7,500 liter fermentor was filled with 4,500 liters of water and charged with 225 kg dextrose, 27 kg yeast extract and 450 grams of antifoam (Dow 1520). The pH was adjusted to 5.0 and the medium was sterilized for 60 minutes at 121°C. After sterilization and cooling to 28°C, the pH was adjusted to 5.5 with NaOH, the aeration adjusted to 0.25 VVM, the back pressure set at 0.2 bar, and the agitation of the A315 impellers was set at a speed of 80 cm per second. The culture was inoculated with 180 liters of a 20 hour old seed culture of *Mortierella alpina* at 2.2 grams per liter. The pH was allowed to fluctuate until 37 hours into the run at which time it was controlled at 6.5. The agitation was increased to 110 cm per second at 26 hours during the peak oxygen demand, but it was returned to 80 cm per second at 32 hours. The time course of this fermentation is shown in Figure 3. At 123 hours the tank was harvested using a Bock basket centrifuge fitted with a 40 micron bag. The material was then dried using a fluid bed drier and extracted with hexane as in Example 2. The fermentation yielded 17 kilograms of a crude oil with an ARA content of 45%.

### Example 6

DHA-enriched oil prepared in accordance with Example 1 or 2 was prepared for oral use by either encapsulating or tableting. Clear sealed gelatin capsules of 1 gram per capsule were prepared by conventional manufacturing methods. Banded gel caps containing one oil or a mixture of oils were prepared by allotting 250 µl of oil in the gel cap bottoms using a positive displacement manifold pipetter. With this method weight accuracy of ±3-5% was attained. The tops then were placed over the gels caps and were banded with dyed gelatin using a capsule banding machine. Similarly, the oil can be encapsulated in soft gelatin capsules using standard commercial technology. Alternatively, the gel cap bottoms were filled with carboxymethylcellulose and 120 µl of oil was pipetted directly onto this binder where it was adsorbed, preventing any leakage. The tops were placed over the gel caps and were banded with dyed gelatin using a capsule banding machine. Alternatively, the carboxymethylcellulose could be mixed with oil at a ratio of three parts carboxymethylcellulose to one part oil in a separate container and then pressed into tablets using a tablet press.

The procedure was repeated using ARA-enriched oil produced in accordance with Examples 4 and 5.

### Example 7

Crude microbial oils produced from microorganisms such as those described in examples 1, 2, 3, 4 and 5 were processed using conventional vegetable oil processing methods. The oil was degummed to remove phosphatides by mixing with water at 50°C, then removing the water and gum mixture by centrifugation. The oil was refined to remove the free fatty acids by mixing with caustic base followed by phosphoric acid at 55°C, then removing the water, fatty acid mixture by centrifugation. The processed microbial oil was bleached by mixing with citric acid and standard bleaching clay at 90°C before filtration to remove the spent clay and any remaining polar particles in the oil. In some cases the oil was deodorized using either a high vacuum distillation or a counter current steam stripping deodorizer resulting in the production of the final, refined, bleached and deodorized oil (RBD oil). Specifications of the oils flowing through this process typically gave a peroxide value of less than 2 mEq per kilogram and a free fatty acid level of less that 0.5 %. These specifications are typical for standard vegetable oils and the microbial oils are used in this state in place of vegetable oil in the preparation of margarine, shortenings, spoonable dressings, liquid dressings, or as the oil component of other manufactured food products. The microbial oils are highly enriched in long chain polyunsaturated fatty acids, in particular DHA and ARA, and are diluted by at least one part per ten parts of a conventional oil chosen for the particular product being prepared. For incorporation into chocolate products, the oil is diluted with cocoa butter. For use as a shortening or baking product, the oil is diluted with coconut or palm oils. For use as a salad dressing, the oil is diluted with standard salad oils such as canola, soy, safflower or corn oil.

### Example 8

A patient exhibiting the symptoms of Alzheimer's disease is treated with DHA-enriched oil from *C. cohnii* by administering 1 to 3 capsules containing 1 gram of DHA single cell microbial oil, containing 50% DHA, per day. The patient's serum levels of DHA and plasmalogens are routinely monitored during the period of the administration in order to determine when the serum levels of these two substances are normalized. Serum DHA levels of two-times the American normal are preferred.

### Example 9

A patient with a peroxisomal disorder was administered 0.5 ml (500mg) of DHA oil at a concentration of 200-250 mg of DHA, directly into the gasmostomy feedings consisting of Sustical (Ross Laboratories) once a day. The patient's serum DHA and plasmalogen levels were routinely monitored during the period of administration. Within six weeks of the initiation of the treatment, the patient's DHA levels improved from 1.85 µg/ml of plasma to 13.6 µg/ml of plasma. Normal levels are 19.0 ± 6.4 µg/ml. Unexpectedly the patient's plasmalogen levels were normalized as well. With the treatment of the DHA oil alone, however the serum ARA-levels remained relatively unchanged at about 50 % of normal levels.

### Example 10

A patient suffering from a form of senile dementia whose serum DHA and ARA levels are depressed is administered 1 to 3 one gram capsules per day, each of which contains DHA and ARA oils at a ratio of about 2:1 ARA to DHA and an overall dose of DHA of 500 mg per day and ARA of 1000 mg per day. The patient's serum lipids are rechecked in four weeks. If the serum DHA and ARA levels are less than five times the normal levels (i.e., if serum DHA is less than 100 µg per ml of plasma and serum ARA less than 500 µg per ml plasma), and the symptoms persist, the patient should remain on the same dose regimen until the serum DHA and ARA reach the desired levels and/or symptoms are alleviated. The dose then can be lowered until the symptoms once again appear or until the serum ARA and DHA levels are in the normal range.

### Example 11

A patient with schizophrenia is administered 1 to 9 one gram capsules per day, each of which contains a balance of DHA and ARA oils providing an ARA/DHA ratio of about 2:1 and an overall dose of DHA of 1000 mg/day and ARA of 2000 mg/d. The patient's serum lipids are rechecked in four weeks. If the serum DHA and ARA levels are less than five times normal levels (i.e., DHA less than 100 µg/ml plasma and ARA less than 500 µg/ml plasma) and the symptoms persist the patient should remain on the same dose regimen until the desired levels are reached and/or symptoms are alleviated. Once the symptoms of the neuropathy are relieved at the given dose, the maintenance dose can be titrated down until the symptoms once again appear or until the serum ARA and DHA levels are in the normal range.

### Example 12

Blood lead levels of greater than 10 µg per deciliter are considered "neurologically significant" by the U.S. Centers for Disease Control and Prevention. Patients tested and found to have blood lead levels in excess of 10 µg per deciliter are administered 1-3 one gram gel capsules per day, each of which contains a balance of DHA and ARA oils providing an ARA/DHA ratio of about 2:1 and an overall dose of DHA of 250 mg/day and ARA of 500 mg/d. The patient's serum lead levels, plasma fatty acids and plasmalogen levels are rechecked in four weeks. If the serum DHA and ARA levels are less than five times the normal levels (i.e., DHA less than 100 µg/ml plasma and ARA less than 500 µL/ml plasma) and the symptoms or elevated lead levels persist, the patient should remain on the same dose regimen until the desired fatty acid levels are achieved and/or the symptoms are alleviated. Once the serum ARA and DHA levels are greater than five times the normal level then the dose levels should be reduced. If the symptoms of the neuropathy are relieved or the serum lead levels are reduced at the given dose, then the maintenance dose may be titrated down until the symptoms once again appear or until the serum ARA and DHA levels are in the normal range.

### Example 13 DHA Therapy for Peroxisomal Disorders

Four patients with generalized peroxisomal disorders (two siblings with Infantile Refsum Disease aged 19 and 23 years, a 12 year old patient with neonatal adrenoleukodystrophy and a 6 month old infant with Infantile Refsum Disease) were supplemented for periods ranging from one to two years with DHA-containing triglyceride derived from microalgae (100-150 mg/kg/day). Fatty acid content at various times, and clinical parameters after DHA levels were normalized are shown in Tables 13-1 and 13-2, respectively. The erythrocyte membrane and plasma phospholipid concentrations of DHA were normalized in all patients as a result of this treatment. Slight improvement of the clinical condition and ERG maximal amplitude was seen in two patients and no further neurodegeneration was observed in the remaining two patients.

**Table 13-1. Fatty Acid Content In Plasma**

| Patients Age | Period of Supplementation (days) | DHA** | Arachidonic Acid |
|---|---|---|---|
| 12 years | 0 | 6.43 | 46.55 |
| | 149 | 13.28 | 63.83 |
| | 294 | 22.18 | 66.75 |
| 6 months | 0 | 5.85 | 59.72 |
| | 108 | 44* | 67* |
| | 313 | 43.30 | 54.39 |
| 23 years | 0 | 7.85 | 44.71 |
| | 132 | 10.06 | 53.24 |
| | 273 | 11.32 | 38.47 |
| 19 years | 0 | 4.27 | 45.33 |
| | 144 | 9.40 | 54.55 |
| | 281 | 14.76 | 43.05 |
| | 504 | 24.68 | 65.39 |

| | | | |
|---|---|---|---|
| * in Red Blood Cells ** DHA, C22:6ω3 (normal = 36.6±5.0 nm/10⁹) *** Arachidonic Acid, C20:4ω6 (normal = 150.0±30.0 mm/10⁹) | | | |

**Table 13-2. Clinical Parameters**

| Patient's Age | Clinical Examination | IRM | ERG |
|---|---|---|---|
| 12 years | no change | cerebellar hypoplasia | Flat |
| 6 months | improvement | normal | no change |
| 23 years | improvement | cerebellar hypoplasia | no change |
| 19 years | no change | cerebellar hypoplasia | no change |

### Example 14 Effect of Aging on Cognition and Fatty Acid Composition in Brain

Young Han:Wistar male rats (n=10, 3.5-4 month old) and aged Han:Wistar male rats (n=12, 24.5 month old), not previously used in any other experiments, were singly housed in a controlled environment (temperature 22 ± 2 °C, humidity 50-70%, light on from 7:00 am to 9:00 pm). Food and water were available *ad libitum.*

### EEG Recordings

Some of the animals were anesthetized with chloral hydrate (350 mg/kg i.p.) and placed in a stereotaxic frame while active recording electrodes were implanted. EEG measurements were taken in recording cages while the rats moved freely. Three 30 min. cumulative walking-immobility (eyes open, head held up) recordings were made on consecutive days. Recordings were made during the same time of the various recording days for individual animals. The IBM-compatible software separated high-voltage spindles from background EEG and counted the total duration (seconds) of high-voltage spindles from the right active recording electrode during a 30 min. cumulative waking-immobility period. The recording system was also used to collect EEG samples for 1-20 Hz EEG spectral power (microvolts) analysis.

1-20 Hz sum amplitude EEG values show age-related defects of sum amplitude values. High voltage spindling (second) is markedly increased in aged rats. Aged rats had significantly higher high voltage spindle activity (p=0.0002) and lower amplitude values (p=0.0031) than young rats.

### Behavioral Testing

Behavioral testing demonstrated significant age-related learning and memory deficiencies.

### Water Maze

Rats were placed in the water, with their nose facing the wall, at one of the starting points in a random manner. The starting locations which were labelled north, south, east and west were located arbitrarily on the pool rim. The timing of the latency to find the submerged platform was started and ended by the experimenter. Testing consisted of 4 consecutive days of testing: 50 s platform trials were assessed (6 trials per day). On each trial, the rats were allowed to stay on the platform for 10 seconds(s). A 30-s recovery period was allowed between the training trials. The location of the platform was changed every day. The computer calculated the total distance swum (in cm) and the distance (the higher total distance, the worse spatial acquisition performance) in all quadrants (n=4) and annuli (n=3) separately. The relative distance swum in the outer annuli was also calculated as during aging the rats spend a greater proportion of the time searching the platform from the outer annulus (wrong annulus). Escape distance values (cm) during training days 1-4 showed age-related defects in spatial navigation. Aged rats had markedly higher escape distance values during all of the training days (p=0.0011), and aged rats spent more time in the wrong annulus (that which did not contain the escape platform) compared to young rats (p=0.0004: old = 62.5% vs. young = 37.5%).

### Passive Avoidance

Passive avoidance training was started 24 h after the last water maze testing session. Passive avoidance box had a light and a dark compartment. During the training trial, the rats were placed on the light compartment. Thirty s later the sliding guillotine door was opened. After the rat entered the dark compartment the door was closed and a foot shock of 1.0 mA (3 s) was given. During the memory trial 72 h later the rat was again placed in the bright compartment and the latency to enter the dark compartment was measured (360 s maximum latency). Passive avoidance entry latency values were shorter in aged rats (p=0.0001).

### Fatty Acids

After the experiments, the frontal cortex and hippocampi was dissected and stored at -73 °C until assayed for fatty acid content. Liver and heart fatty acid content (shown in Table 14-1) did not differ between young and aged rats. Fatty acid content in brain samples is shown in Table 14-2. Cortical 16:0 level was slightly lower in aged rats than young rats (p = 0.028) and cortical DHA levels were also lower in frontal cortex of aged rats (p = 0.089). Hippocampal 16:0 and 22:6 DHA levels were lower in aged than young rats (p = 0.48 and p = 0.41, respectively). On the contrary, 18:1 n-7 and n-9 levels were higher in the hippocampal samples of aged rats (p = 0.0009 and p = 0.005, respectively).

**Table 14-1. Fatty Acid Content of Peripheral Samples**

| | | | | |
|---|---|---|---|---|
| | | | | |

| | Young | Old | Old | Young |
|---|---|---|---|---|
| 12:0 | 0.7 | 0.1 | - | - |
| 14:0 | 0.7±0.2 | 0.9±0.3 | 0.4±0.1 | 0.4±.01 |
| 16:0 | 12.5±1.5 | 12.8±1.1 | 19.9±1.8 | 20.±0.9 |
| 16:2 | - | - | 01.0±0 | 0.1±0 |
| 17:0 | 0.2±0.1 | 0.2±0.1 | 0.5±0 | 0.5±0 |
| 18:0 | 11.5±2.4 | 9.0±1.9 | 15.6±1.6 | 16.2±1.2 |
| 18:1 n-9 | 5.7±2.0 | 5.7±1.3 | 7.1±0.4 | 7.1±1.1 |
| 18:1 n-7 | 3.5±0.5 | 3.5±0.4 | 4.5±0.4 | 4.6±0.6 |
| 18:2 | 29.1±2.3 | 31.4±1.7 | 16.3±0.8 | 15.9±0.5 |
| 18:3 n-3 | 0.5±0.2 | 0.6±0.2 | 0.3±0.1 | 0.3±0.1 |
| 18:3 n-6 | 0.1±0.1 | 0.0 | 0.2±0.1 | 0.2±0.1 |
| 20:1 | 0.1±0.1 | 0.1±0.1 | 0.3±0 | 0.3±0.1 |
| 20:2 | 0.1±0.1 | 0.0 | 0.3±0.1 | 0.2±0.1 |
| 20:3 n-6 | 0.4±0.1 | 0.3±0.2 | 1.3±0.3 | 1.3±0.3 |
| 20:4 ARA | 18.1±2.9 | 18.0±2.0 | 19.7±1.7 | 19.8±1.6 |
| 20:6DHA | 12.5±2.7 | 12.2±2.2 | 7.6±1 | 7.0±0.8 |
| Unknown | 3.3±0.3 | 2.9±0.4 | 5±0.8 | 4.6±0.3 |

**Table 14-2. Fatty Acid Content of Brain Samples**

| | | | | |
|---|---|---|---|---|
| | | | | |

| | Old | Young | Old | Young |
|---|---|---|---|---|
| 16:0 | 21.2+0.7 | 22.2+0.6 | 21.5+0.8 | 22.2+2.3 |
| 16:1 | 0.1+0.3 | 0.2+0.3 | 0 | 0.1+0.2 |
| 18:0 | 18.7+1.5 | 19.2+0.6 | 19.1+1.1 | 18.9+1.7 |
| 18:1 n-9 | 19.9+1.9 | 19.1+1.1 | 20.6+1.1 | 18.5+1.6 |
| 18:1 n-7 | 3.5+0.4 | 3.4+0.2 | 3.7+0.2 | 1.9+1.6 |
| 18:2 | 0.3+0.3 | 0.1 +0.2 | 0+0 | 0.2+0.7 |
| 20:1 | 0.2+0.7 | 0.5+0.9 | | |
| 20:2 | 0.3+0 | 0+0 | 0+0 | 0.3+1.1 |
| 20:4 ARA | 13.3+0.5 | 14.1+1.3 | 15.1+0.5 | 15.8+2.7 |
| 22:4 | 3.1+0.3 | 3.4+0.3 | 3.5+0.3 | 2.5+1.7 |
| 22:6 DHA | 16.5+1.1 | 17.5+1.4 | 15.3+0.7 | 17.5+3 |
| Unknown | 2.9+3.7 | 0.1 +0.3 | 1.2+2.6 | 2.1 +6.8 |

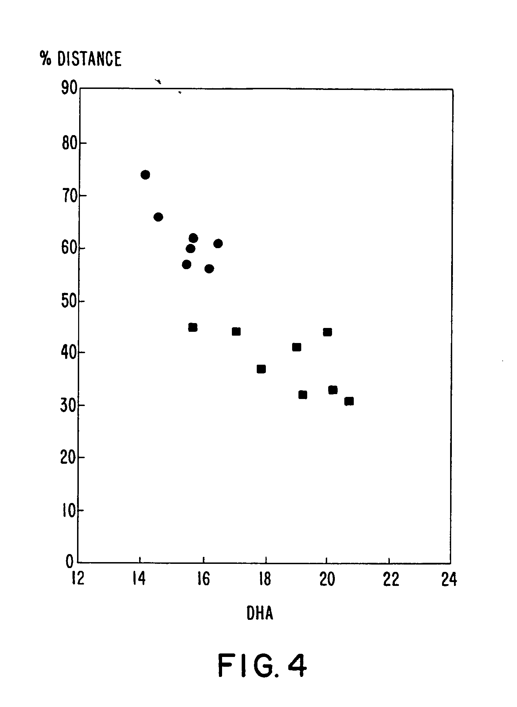

The present results indicate that the content of fatty acids is unaltered in liver and heart in Han:Wistar rats fed with a regular diet. However, brain DHA content was decreased in both the cortex (6%) and hippocampus (13%) in aged rats. The correlation of hippocampal DHA with cognitive performance (Figure 4) indicates an adverse effect of lower DHA levels. Figure 4 shows that the time spent in the wrong annulus correlated with hippocampal DHA content (r=-0.8927, p < 0.001).

The results showing that only brain DHA content (not liver or heart) was decreased may suggest that the synthesis of DHA by astrocytes (Moore, et al., 1991, J. Neurochem. , 56:518) is decreased during ageing while peripheral production of DHA is relatively intact in aged rats. Therefore, it is possible that the impaired DHA metabolism may, via dysregulation of NMDA receptor-mediated mechanism, impair synaptic plasticity and memory functioning (Fujimoto, et al., 1989, in Health Effects of Fish and Fish Oils, p. 275; Nishikawa, et al., 1994, J. Neurophys., p. 83).

### Example 15 Tissue Distribution of HUFA in Rats

### Supplemented with DHA- and ARA- Containing Oils

Rats were orally gavaged for 4 weeks with 3,750mg/kg/d high oleic sunflower oil, or an equal amount of mixed oil in which a portion of the high oleic sunflower oils was replaced with 50mg/kg/d ARASCO (high ARA oil from fungal fermentation), 1,000mg/kg/d ARASCO, 2,500mg/kg/d ARASCO, 25mg/kg/d DHASCO (high DHA oil from algal fermentation), 500mg/kg/d DHASCO, 1,250mg/kg/d DHASCO, 1,500mg/kg/d Formulaid, or 3,750mg/kg/d Formulaid. (Formulaid is a 1:2 mixture of DHASCO:ARASCO).

The fatty acid content of various rat tissues after the four week feeding period are shown in Tables 15-1, 15-2 and 15-3. Those marked with asterisks differ significantly (two-tailed Student's t-test) from the control values.

### Example 16 Arachidonic Acid Supplementation Study

Oil containing 1.2 grams of arachidonic acid per day were fed for fifty days to twelve healthy subjects (3.0 grams/day of ARASCO oil containing ARA in triglyceride form; 1.2g arachidonic acid/d). The natural foods consumed by the volunteers during the studies provided another 300 mg/day of arachidonic acid.

There were no adverse effects on the health of the volunteers during the study. They were examined before and after the intervention period by a physician who declared them to be in excellent health, (i.e., no manifestations of clinical disorders on the macroscopic level). Their blood work showed all values within the normal range. Using research laboratory tests, no changes were observed in platelet aggregation in PRP with ADP, collagen, or arachidonic acid. Lipoprotein and blood cholesterol levels were essentially unchanged during the study. There was no evidence of change in the immune responses of these volunteers, nor were their immune functions suppressed or enhanced. Adipose tissue levels of arachidonic acid in these subjects did not increase after consuming the arachidonic acid diet. Significant increases in the plasma levels of arachidonic acid were found when the subjects were fed the arachidonic acid diet.

### Example 17 DHA Supplementation Study

This study examined the effect of supplementing the diet of healthy subjects with DHASCO capsules (six 500 mg capsules per day, each containing about 47% DHA in triglyceride form, but no other HUFAs) versus placebo capsules (six 500 mg capsules per day containing no DHA). Eighteen normolipidemic subjects (ages 18-49, both sexes) with normal renal, kidney, and thyroid function on normal *ad libitum* diets were randomized in a blinded fashion to the DHASCO capsules or placebo for a five month period. During the course of this study, approved by the Human Investigation Research Committee, no adverse effects of any kind were noted, including no effects on standard blood chemistries and complete blood counts.

Analysis of plasma lipids, lipoproteins, apolipoproteins, plasma fatty acids, red blood cell (RBC) phosphatidylethanolamine (PE) fatty acids, and various tests of immunologic function have been performed, and the data is shown in Tables 17-1, 17-2, 17-3 and 17-4. There was a statistically significant increase in HDL and ApoAl using a paired t-test for comparison of before and after treatment samples (Table 17-1). Although there was a 20% decrease in serum triglycerides (Table 17-1) in the DHASCO-supplemented group, the sample size was too small to show significance at the 95 % level.

Meydani, et al., (1994, J. Clin. Invest., 92:105-113) reported that a fish oil diet affected a number of immune system parameters including lymphocyte proliferation (decreased relative to controls) leukocyte CD4⁺ counts (decreased), and leucocyte CD8⁺ counts (increased), while total T lymphocyte counts were unchanged. Similar tests on the subjects fed DHASCO or placebo in this study (Table 17-2) showed no change in CD4⁺ and CD8⁺ leukocytes or in lymphocyte proliferation and a small increase in total T cells. No significant changes in any of the other parameters at 2 and 3 months versus baseline were observed in either the placebo or DHASCO capsule group, except for two to threefold increases in DHA content of phosphatidylethanolamine (PE) of plasma (Table 17-3) and RBC (Table 17-4) for the DHASCO group (p<0.0001).

**Table 17-1. DHA Supplementation Study: Lipid Data**

| | **Placebo Group (n=9)** | | **DHASCO Treatment Group (n=9)** | |
|---|---|---|---|---|
| | **Baseline** | **Treatment (of Mos. 2&3)** | **Baseline** | **Treatment (of Mos. 2&3)** |
| TC | 191.8±40.7 | 199.6±40.1 | 215.7±34.5 | 222.0±31.3 |
| TG | 89.8±35.0 | 99.9±59.6 | 194.3±133.2 | 155.5±78.7 |
| HDL | 36.6± 9.7 | 38.3±10.5 | 29.8± 10.7 | 32.4±11.2* |
| LDL | 137.3±39.0 | 141.4±35.6 | 147.0±22.0 | 152.8±24.4 |
| ApoA1 | 139.2±19.8 | 145.t±23.6 | 120.3 ±26.39 | 126.9±24.4* |
| ApoB | 93.67±27.9 | 94.72±24.7 | 107.6±18.4 | 108.6±18.2 |
| Lp(a) | 22.0±18.1 | 20.9±16.3 | 10.3±12.1 | 11.9±15.3 |

| | | | | |
|---|---|---|---|---|
| All values are mean ± SD paired t-Test: * p <0.05 | | | | |

**Table 17-2. DHA Supplementation Study T-Cell Characteristics: Immunology Study"**

| | **Placebo Group (n =9)** | | **Treatment Group (n=9)** | |
|---|---|---|---|---|
| | **Baseline** | **Post-Treat** | **Baseline** | **Post-Treat** |
| LPP1^{b} | 2220±1359.8 | 5238.3±4031.2 | 2013±1617 | 5780±6657 |
| LPP10^{b} | 56998.7±15462.4 | 56424.6±22192.4 | 56412±21703.9 | 64340±45110 |
| LPP100^{b} | 30439±13694 | 31567.3±2034.3 | 31482±20625 | 37103±38641 |
| LPC10^{b} | 2901.5±1490 | 6448.5±3945.5* | 1690±1205 | 3541±2552* |
| LPC50^{b} | 14669.5±4085.2 | 20932.8±11530.3 | 9230.4±5445.4 | 20336±17586 |
| LPC100^{b} | 22116.5±9803.5 | 22890.1±18283.6 | 18309.3±10079.8 | 26863±26166 |
| LEU3A^{c} | 15.13±6.8 | 49.53±7.3 | 49.95±12.01 | 51.23±11.86 |
| LEU4^{d} | 76.9±7.5 | 75.7±8.5 | 74.08±12.01 | 76.5±9.7* |
| LEU2A^{c} | 21.32±6.3 | 20.32±4.8 | 19.86±5.7 | 21.42±7.45 |

| | | | | |
|---|---|---|---|---|
| All values are mean ± SD paired t-Test: * p <0.05 ** p <0.01 *** p <0.001 a Method described in Meydani, et al., 1994, J. Clin. Invest. 92:105-113 b Lymphocyte Proliferation c CD4⁺ Leukocytes d Total T Cells | | | | |

**Table 17-3. DHA Supplementation Study: Percentage of Plasma Fatty Acids**

| | **Placebo Group (n=9)** | | **Treatment Group (n=9)** | |
|---|---|---|---|---|
| **FA** | **Baseline** | **Post-Treat** | **Baseline** | **Post-Treat** |
| 16:0 | 23.1±2.1 | 23.3±1.2 | 22.9±1.8 | 23.4±4.6 |
| 16:1 n-7 | 3.0±.9 | 3.20±.92 | 3.4±.9 | 3.1±.8 |
| 18:0 | 10.1±1.5 | 11.2±1.9 | 13.7±3.6 | 12.6±4.8 |
| 18:1 n-9 | 21.9±2.8 | 21.3±3.2 | 21.5±4.9 | 20.3±4.1 |
| 18:2 n-6 | 26.2±1.5 | 25.9±2.7 | 24.2±4.6 | 23.6±3.2 |
| 18:3 n-6 | 0.56±.18 | 0.62±.3 | 0.79±0.6 | 0.68.3 |
| 18:3 n-3 | 0.44±1.9 | 0.53±.2 | 0.49±.2 | 0.42±.1 |
| 20:3 n-6 | 21.±1.4 | 2.3±.5 | 1.9±.23 | 1.69±.2* |
| 20:4 n-6 | 8.9±1.7 | 8.6±1.8 | 7.9±2.1 | 6.7±1.5** |
| 20:5 n-3 | 0.64±.5 | 0.47±.2 | 0.76±.9 | 0.79±.5 |
| 20:5 n-3 | 0.76±.2 | 0.75±.2 | 0.74±.2 | 0.50±.15** |
| 20:6 n-3 | 2.1±.9 | 1.9±.7 | 1.9±.6 | 5.9±1.5*** |

| | | | | |
|---|---|---|---|---|
| All values are mean ± SD paired t-Test: * p < 0.05 ** p<0.01 | | | | |

**Table 17-4. DHA Supplementation Study: Percentage of RBC Fatty Acids (Treatment Group Only)**

| | Baseline | Post-Treatment |
|---|---|---|
| DMA 16:0 | 5.13±.35 | 4.70±.63 |
| 16:0 | 14.94±1.5 | 16.24±1.6** |
| DMA 18:0 | 10.07±9.1 | 9.58±.89 |
| DMA 18:1 n-6 | 3.97±.55 | 3.84±.85 |
| 18:0 | 7.12±.54 | 6.98±.66 |
| 18:1 n-9 | 17.01±1.1 | 17.6±1.2 |
| 18:2 n-6 | 5.66±1.1 | 5.48±1.2 |
| 20:3 n-6 | 0.91±1.6 | 0.84±.10* |
| 20:4 n-6 | 19.7±1.0 | 17.62±1.1*** |
| 20:5 n-3 | 0.69±.22 | 0.82±.31 |
| 22:4 n-6 | 6.21±.62 | 4.53±.53*** |
| 22:5 n-6 | 0.72±.11 | 0.45±.08*** |
| 22:5 n-3 | 3.04±.41 | 1.97±.25*** |
| 22:6 n-3 | 4.88±1.77 | 9.46±1.8*** |

| | | |
|---|---|---|
| Paired t-Test: * p < 0.05 ** p < 0.01 *** p < 0.001 | | |

### Example 18 Study of Dose Response to DHA Supplementation

A group of 17 subjects were studied as described in Example 17, except diets were supplemented with one-half the amount of DHA-containing oil as Example 15 (i.e., 1.5g DHASCO/day). Table 18-1 and 18-2 show the effect on DHA and ARA levels in plasma and red blood cell phosphatidyl ethanolamine, respectively. The baseline (pre-treatment) values differ from Example 15 due to differences in the analytical procedure. The study shows that supplementation with this amount of DHA results in a 220% increase in plasma phospholipid DHA and an 8.7% decrease in arachidonic acid level.

**Table 18-1: Percentage Fatty Acid Content in Plasma at Lower DHA Supplementation**

| **DHA And ARA As Percent of Total Plasma PE Fatty Acids (Mean ± SD)** | | | | |
|---|---|---|---|---|
| | DHA | | Arachidonic Acid | |
| | DHA Group (n=9) | Placebo Group (n=8) | DHA Group (n=9) | Placebo Group (n=8) |
| Pre-Treatment | 0.94±0.59 | 0.96±0.34 | 5.51±0.47 | 6.64±1.9 |
| 3 months Post-Treatment | 2.10±0.54 | 0.85±0.32 | 5.03±0.85 | 6.20±1.7 |

**Table 18-2. DHA And ARA as Percent of Total Fatty Acid Content of Red Blood Cell PE**

| **Percent of Total RBC PE Fatty Acids (Mean ± SD)** | | | | |
|---|---|---|---|---|
| | DHA | | Arachidonic Acid | |
| | DHA Group (n=9) | Placebo Group (n=8) | DHA Group (n=9) | Placebo Group (n=8) |
| Pre-Treatment | 3.37±1.19 | 3.92±0.98 | 13.66±0.96 | 14.53±1.49 |
| 3 months Post-Treatment | 6.52±1.74 | 4.12±0.96 | 12.69±0.93 | 14.80±1.19 |

### Example 19 Dose Study of DHA-containing Oil in Subjects With Type IIB by Hyperlipidemia

This study was a randomized, double-blind, placebo controlled study of DHA supplementation using three parallel groups involving subjects with mild hypercholesterolemia (LDL-C=130-220 mg/dL). The desired sample size was 9 subjects in each group, for a total evaluable sample of 27 subjects. Each subject underwent a complete medical history and physical examination, and demonstrated no evidence of any chronic illness including endocrine, hepatic, renal, thyroid, cardiac dysfunction, drug abuse or known allergy to fish or fish oil (see Table 19-1). Post-menopausal women were included in the study. Concomitant use of lipid-lowering medications and therapies were discontinued for at least four weeks prior to the first qualifying blood draw. Subjects who successfully completed the initial 6-week screening/dietary phase entered the 4-week placebo lead-in phase.

Plasma lipid eligibility (LDL-C 130-220 mg/dL and triglycerides 150-400 mg/dL) was determined by the mean of two consecutive lipid profile measurements obtained at Week -2 and Week -1. An individual coefficient of variation of ≤ 15 % for these lipid values was set, *a priori,* as a criterion for inclusion. Laboratory measurements, including lipid profiles (total cholesterol, triglycerides, HDL-C AND LDL-C), were determined. LDL-C levels were calculated using the Friedewald formula (Friedewald, et al., 1972, "Estimation of LDL-Cholesterel in Plasma Without Use of the Preoperative Ultracentrifuge," Clin. Chem. , 18:499). All lipid laboratory tests were standardized against the Center for Disease Control lipid standardization program.

Following the screening/dietary and placebo run-in phases, eligible patients were randomly assigned to one of the three study groups and given their appropriate supplements under double-blind conditions. Subjects who were at least 80 % compliant during the placebo lead-in phase were randomly assigned to receive gelatin capsules containing 500 mg of triglyceride having 48 % DHA at levels of either 6g DHASCO (12 DHASCO capsules/day), 3.0g DHASCO (6 DHASCO capsules and 6 placebo capsules) or 0g DHASCO (12 placebo capsules containing a 1:1 mixture of corn and soy oil) for 6 weeks.

All subjects were required to adhere to the 30% or less fat recommendations of the National Cholesterol Education Program Step I diet throughout the trial. Adherence was monitored throughout the study utilizing food records evaluated by computerized nutrient analysis. Patients returned to the clinic at three week intervals for vital signs, weight, dietary counseling and a compliance check. Blood samples were obtained for monitoring lipid profiles at each visit during the treatment phase. Safety parameters (including serum chemistry, hematology and urinalysis) were also evaluated while subjects were on treatment. Adverse effects and concomitant medications were reported and documented throughout the study.

Mixed factor analysis of variance was performed to test for possible treatment and time effects, and treatment-time interaction for lipid, body weight and compliance data. When significant F-ratios were found, this was followed by Scheff's procedure for multiple comparisons. Separate analysis of covariance models were also run using the baseline lipid values as covariates, but these results did not differ from those generated by analysis of variance, so only the latter are shown. Spearman rank correlation coefficients were calculated as a measure of the degree of association between the changes in different lipid values during treatment. Treatment values are determined as the means of Week 3 and Week 6.

Of the 27 subjects randomized, 26 completed the study. All subjects met the inclusion criteria per protocol guidelines except for nine subjects who deviated from the protocol for either age or body mass index. Separate analyses were performed on the data with these subjects excluded. Since the analyses produced results consistent with the primary analyses, these subjects are included in the final results.

Overall, the subjects found the capsules very tolerable and there were virtually no side effects. There were no adverse events observed during the course of the study. No significant differences were found between groups with regard to age, race, sex, BMI, percent ideal body weight or serum lipids. Compared to baseline, there were no differences observed between or within groups for energy intake, percent of caloric intake from carbohydrates, total fat, saturated fatty acids, polyunsaturated fatty acids, monounsaturated fatty acids, or alcohol. There was a nonsignificant increase in mean body weight after six weeks of treatment in the placebo (0.69±1.61 kg), 3.0g DHASCO (1.17±0.79 kg), 6.0g DHASCO (1.25±1.10kg) groups, respectively.

The results for various lipid variables are shown in Table 19-2. Despite significant changes over time for some lipid variables, group means did not differ during treatment. Considering the relatively small sample studied, this is not surprising since the statistical power was below 40 % for detecting a difference as large as one standard deviation.

There was no significant change in total cholesterol among the three groups during treatment. There was no significant change in LDL-C in the placebo and 3.0g DHASCO groups; however, those taking 6.0g DHASCO showed a mean increase in LDL-C concentration of 13.6% (<0.01). There was a significant (p < 0.05) increase in the HDL-C in both DHA treatment groups, but not in the placebo group.

DHA supplementation was associated with significant reductions in triglycerides for both the 3.0g (-20.9%) and 6.0g (-17.6%) DHASCO groups (p=0.01 for each). No alteration was observed within the placebo group. The F-value for treatment effect approached, but did not reach statistical significance (p=0.08). Relationships between the changes in serum lipid parameters among subjects taking DHA supplements were tested using Spearman ranked correlation. The change in triglyceride concentration was significantly associated with change in HDL-C (rs= -0.47, p= 0.05), but not with the change in LDL-C (rs= 0.08). Changes in total cholesterol were highly correlated with changes in LDL-C (rs= 0.90, p= 0.0002), but not with changes in HDL-C (rs= 0.18) or triglycerides (rs= 0.35).

The doses of DHA used in this investigation were well tolerated and had sustained hypotriglyceridemic effects. The findings indicate that daily supplementation with an algae-derived DHA-rich oil resulted in significant reductions in serum triglyceride concentration (18-20%) among persons with Type IIb dislipidemia. HDL-C also rose by a small, but significant amount during DHA supplementation, and the increase was significantly associated with the degree of triglyceride reduction. A 10.0% increase in LDL-C was observed in the high dose DHA group; the pattern observed suggests a possible dose-response effect.

This study was limited to subjects with a single lipoprotein phenotype (IIb). The low dose supplements provided in this study contained no cholesterol and very small amounts of saturated fatty acids. Dietary variability was minimized by including a diet stabilization period in the design. The results suggest that hypotriglyceridemic effects of supplements with algae-derived DHA-containing triglycerides are similar in magnitude to those produced by combined EPA/DHA (fish oil) supplements (Davidson, et al., 1991; Harris, et al., 1990, "Fish Oils in Hypertriglyceridemia: A dose-Response Study," Am. J. Clin. Nutr., 51:399-406; Radix, et al., 1990, "N-3 Fatty Acid Effects on Lipids, Lipoproteins, and Apolipoproteins at Very Low Doses: Results of a Randomized Controlled Trial in Hypertriglyceridemic Subjects," Am. J. Clin,. Nutr., 51:599-605). Reduction in triglycerides per gram of marine oil observed previously among Type IIb dyslipidemic subjects was 5.0-9.0% compared to 7.0-12% reduction in the present study (Radix, et al., 1990). HDL-C also rose by 7. 8-9.5 % as compared to 6 % in the present investigation. Because of the high DHA content of the algae-derived DHA oil, 1g of DHASCO is equivalent to 5-6g of fish oil.

Radack, et al. (Radack, et al., 1990) found that 2.2g of fish oil per day produced a statistically significant increase of 28% in LDL-C among hypertriglyceridemic patients (mixed phenotype), while lowering triglycerides only marginally (~16%). Harris, et al (Harris, et al., 1990) found that the LDL-C increased progressively from 8-28% with increasing doses of fish oil containing 4.5-12.0g per day of omega-3 fatty acids. (LDL-C rose by 8% with the lowest dose and 28% with the highest dose). In the present study, LDL-C increased by 13.6±2.6% in the group receiving 3.0g DHASCO per day. Thus, our findings are not consistent with the postulate that EPA is entirely responsible for the LDL-C raising properties of marine oils. Nevertheless, omega-3 fatty acids have numerous potentially antiatherogenic effects irrespective of their influence on plasma lipids (Kinsella, et al., 1990, "Dietary n-3 Fatty Acids and Amelioration of Cardiovascular Disease: Possible Mechanisms," Am. J. Clin. Nutr., 52:1-28), and the net effect may prove to be beneficial despite some degree of LDL-C elevation. This argument is strengthened by results of a randomized secondary prevention trial which found significant reductions in mortality among persons consuming diets enriched in omega-3 fatty acids (McKeigue, P., 1994, "Diets for Secondary Prevention of Coronary Heart Disease: Can Linolenic Acid Substitute for Oily Fish?" Lancet, 343:1445).

In summary, low dose supplementation of algae-derived DHASCO (3.0g/d; 6.0g/d) resulted in a clinically significant decrease of triglycerides, no significant change in total cholesterol or LDL-C, and a small, but significant, rise in HDL-C.

**Table 19-1: Demographics**

| | Placebo n=8 | 1.5g n=9 | 3.0g n=9 |
|---|---|---|---|
| Age (Mean) | 57 | 61 | 58 |
| SEX Male | 4 | 6 | 8 |
| Female | 4 | 3 | 1 |
| RACE White | 7 | 7 | 9 |
| Black | 1 | 1 | 1 |
| Asian | 0 | 1 | 0 |
| BMI (baseline) | 28.5 | 29.3 | 27.8 |
| % Ideal Body Wt. (baseline) | 130 | 132 | 128 |
| Serum T-Chol | 252.7 | 253.1 | 270.6 |
| Lipids LDL-C | 162.0 | 168.8 | 179.7 |
| HDL-C | 45.4 | 46.1 | 42.8 |
| Triglyc. | 226.0 | 190.4 | 244.8 |

**Table 9-2: % Change From Baseline for Lipid Values**

| | Placebo | | 3.0g DHASCO | | 6.0g DHASCO | |
|---|---|---|---|---|---|---|
| | % Change | p-value | % Change | p-value | % Change | p-value |
| Total Cholesterol | +2.0% | ns | +1.6% | ns | +5.4% | ns |
| LDL-C | + 2.4% | ns | +9.3% | ns | +13.6% | 0.01 |
| HLD-C | +5.2% | ns | +6.0% | 0.05 | +6.2% | 0.03 |
| Triglycerides | -3.5 % | ns | -20.9% | 0.01 | -17.6% | 0.01 |

### Example 20 Comparison of the Effects of Fish Diet, Fish Oil and DHA-Containing Oil on Fasting and Postprandial Plasma Lipid Levels

This study was planned to clarify the effects of fish diet, fish oil and DHA-containing oil on fasting and postprandial lipid levels. Subjects were healthy male students (n=59). Fish meals were provided at workdays to subjects in the fish diet group for 14 weeks. Fish species used were rainbow trout, Baltic herring and vendace. The amount of meals actually eaten was 4.30±0.5 per week. This provided 0.38±0.04 g EPA and 0.67±0.09 g DHA per day (calculated on the basis of meal sample analyses). Fish oil group ate 4g fish oil per day and it provided about 1.3 g EPA and 0.9 g DHA. DHA-group ate 4g DHASCO per day oil which contained about 42 % DHA. One group served as controls.

Fat tolerance tests were made before and after the dietary period. A cream mixture (0.7 g/kg) with fatty acid composition near the average of the Finnish diet was used as the test meal. Blood samples were collected up to 8 hours after the meal.

Preliminary results (Table 20) show that both fasting plasma triglyceride levels and postprandial triglyceride response were reduced in all test groups. In the fish diet group, plasma triglycerides decreased from 1.36±0.47 to 1.16±0.40 mmol/l. In fish oil and DHA-oil groups, they decreased from 1.21±0.35 to 0.89±0.13 and from 1.17±0.38 to 0.97±0.21 mmol/l, respectively. Most of these changes occurred in VLDL-triglycerides. HDL3-triglycerides were also decreased. Total plasma cholesterol levels were not changed. However, the HDL2/HDL3-cholesterol ratio increased in all test groups by over 50%. The increase in HDL2-cholesterol was greatest in DHA-oil group. LDL-cholesterol showed a slight tendency to increase in fish diet and fish oil but not in the DHA-oil group.

The postprandial triglyceride responses have been measured as areas under the postprandial concentration curves. The increase of total plasma triglycerides (mmol/l x h) was 4.6±1.4 before and 3.3±2.1 after the dietary period in the fish diet group. In the fish oil group these values were 4.3±2.4 and 3.0± 1.2, and in the DHA-oil group 3.7±2.0 and 3.1±1.0. The increase in chylomicron triglyceride concentration followed the same pattern. It decreased from 2.7±1.1 to 2.2±1.4 in the fish diet group, from 2.7±1.7 to 2.0±0.6 in the fish oil group and from 2.4±1.3 to 1.9±0.6 in the DHA-oil group. Chylomicron cholesterol concentrations were also decreased.

These results show that both fasting and postprandial triglyceride concentrations can be decreased with moderate intakes of long-chain n-3 fatty acids either from a fish diet or fish oil. The changes in DHA-oil group were slightly smaller but the results clearly show that also DHA has a hypotriglyceridemic effect. It appears that the antiatherogenic effect of n-3 fatty acids is partly caused by their effects on postprandial lipid metabolism. Altered lipoprotein concentrations may modify also concentrations and activities of other plasma components.

**Table 20: Cholesterol Levels (mmol/l) in Plasma**

| **CONTROLS** | 0 | 4 | 9 | 14 wk |
|---|---|---|---|---|
| **Total** | 4.97±4.06 | 4.87±1.11 | 4.89±1.11 | 4.85±0.91 |
| VLDL | 0.31±.018 | 0.36±0.19 | 0.31±0.16 | 0.37±0.20 |
| LDL | 2.67±0.70 | 2.67±0.78 | 2.61±0.80 | 2.60±0.76 |
| HDL2 | 1.01±0.28 | 0.99±0.27 | 0.93±0.30 | .077±.030 |
| HDL3 | 0.47±0.07 | 0.40±0.09 | 0.39±0.08 | 0.41±0.09 |

| **FISH DIET** | | | | |
|---|---|---|---|---|
| **Total** | 4.65±1.00 | 4.79±0.99 | 4.72±1.01 | 4.80±0.90 |
| VLDL | 0.24±0.09 | 0.28±0.13 | 0.23±0.09 | 0.25±0.11 |
| LDL | 2.34±0.63 | 2.38±0.63 | 2.42±0.59 | 2.56±0.62 |
| HDL2 | 0.92±0.40 | 0.99±0.28 | 1.01±0.32 | 1.02±0.32 |
| HDL3 | 0.43±0.06 | 0.39±0.08 | 0.31±0.09 | 0.33±0.11 |

| **FISH OIL** | | | | |
|---|---|---|---|---|
| **Total** | 4.53±0.97 | 4.76±1.01 | 4.70±0.94 | 4.56±4.08 |
| VLDL | 0.25±0.08 | 0.21±0.06 | 0.22±0.07 | 0.21±0.07 |
| LDL | 2.28±0.71 | 2.64±0.78 | 2.50±0.77 | 2.51±0.79 |
| HDL2 | 1.03±0.36 | 0.97±0.35 | 1.13±0.38 | 1.01±0.38 |
| HDL3 | 0.43±0.09 | 0.35±0.08 | 8.33±0.09 | 0.32±0.11 |

| **DHASCO** | | | | |
|---|---|---|---|---|
| **TOTAL** | 4.55±0.76 | 4.68±0.33 | 4.83±0.70 | 4.65±0.85 |
| VLDL | 0.26±0.13 | 0.21±0.08 | 0.23±0.10 | 0.22±0.10 |
| LDL | 2.49±0.70 | 2.58±0.62 | 2.44±0.61 | 2.42±0.70 |
| HDL2 | 0.89±0.26 | 1.03±0.36 | 1.11±0.35 | 1.06±0.35 |
| HDL3 | 0.41±0.07 | 0.37±0.05 | 0.32±0.07 | 0.33±0.09 |

| **HDL2/HDL 3** | | | | |
|---|---|---|---|---|
| CONTROLS | 2.19±0.62 | 2.58±0.78 | 2.45±0.98 | 2.00±1.03 |
| FISH DIET | 2.20±0.85 | 2.68±0.79 | 3.58±1.25 | 3.69±1.94 |
| FISH OIL | 2.42±0.69 | 2.98±1.28 | 3.56±1.02 | 3.67±2.22 |
| DHASCO | 2.22±0.67 | 2.79±0.94 | 3.53±1.13 | 3.61±2.11 |

### Example 21 Supplementation With An Algae Source of Docosahexaenoic Acid: Effect on Risk Factors For Heart Disease in Vegetarians

The purpose of this study was to investigate the influence of dietary docosahexaenoic acid (DHA, 22:6ω3) on serum/platelet DHA status and risk factors for heart disease in vegetarians. Healthy vegetarians (12 males, 12 females) were asked to consume 9 capsules daily of either DHASCO (total 1.8g DHA) or vegetable oil (placebo) for 42 days. Blood samples were drawn and fasting serum lipids/lipoproteins and various thrombogenic risk factors were determined. Consumption of DHA capsules increased DHA levels in serum phospholipid by 229% (2.58% to 8.48% wt. %) and platelets by 98%. EPA (20:5n-3) levels increased in serum by 155% and platelets by 30%. Arachidonic acid (20:4n-6) levels in serum and platelets decreased moderately during the trial period (DHA group). Levels of these fatty acids did not change in subjects consuming control capsules. Compared with day 0 values, plasma triglyceride levels were found to decrease by 16% on day 42 in subjects who consumed the DHA capsules (p < 0.05), and HDL-cholesterol levels were 16% higher (p < 0.05). There was no change in the LDL- or total-cholesterol/HDL ratio (12% decrease by day 21 and 15% decrease by day 42, p < 0.05). Consumption of this algae source of DHA did not inhibit collagen-induced platelet aggregation, thromboxane production, or alter fibrinogen, Factor VII levels, or serum viscosity. These results indicate that the consumption of 1.8g DHA by vegetarians is readily incorporated into phospholipids; DHA also undergoes retroconversion to EPA. DHA appeared to have beneficial effects on triglyceride, HDL-cholesterol levels, and the total cholesterol : HDL ratio. Other non-conventional risk factors for heart disease as measured did not appear to be modified.

## Claims

1. A method of alleviating adverse effects of a neurological disorder **characterized by** a DHA deficiency-associated pathology which comprises administering to a patient affected by such a disorder a single cell microbial oil comprising DHA in an amount effective to raise the level of circulating DHA in the patient's blood to within the range of about 15 to 100 µg of DHA per ml of plasma.

2. A method in accordance with claim 1, wherein the disorder is a neurodegenerative disorder.

3. A method in accordance with claim 1, wherein the disorder is a peroxisomal disorder.

4. A method in accordance with claim 3, wherein the peroxisomal disorder comprises Zellweger's syndrome, neonatal adrenoleukodystrophy, infantile Refsum disease, hyperpepecolic acidemia, Rhizomelic chondrodysplasia punctata, Zellweger-like syndrome, adrenoleukodystrophy, adrenomyeloneuropathy, acyl-CoA oxidase deficiency, bifunctional protein deficiency, thiolase deficiency, hyperoxaluria type I, acatalasaemia or adult Refsum disease.

5. A method in accordance with claim 2, wherein the neurodegenerative disorder is Alzheimer's disease.

6. A method in accordance with claim 2, wherein the neurodegenerative disorder is Huntington's disease.

7. A method in accordance with claim 1, wherein the disorder is schizophrenia.

8. A method in accordance with claim 1, wherein the disorder is diabetic neuropathy.

9. A method in accordance with claim 1, wherein the disorder is a neuropathy induced by the ingestion of a pro-oxidant heavy metal comprising lead, aluminum or iron.

10. A method in accordance with claim 1, wherein the DHA oil is administered so as to provide DHA at a dosage level of about 50 mg to about 5000 mg DHA per day.

11. A method in accordance with claim 1, wherein the DHA oil is administered so as to provide DHA at a dosage level of about 500 mg per day.

12. A method in accordance with claim 1, wherein said oil comprises at least 20% DHA.

13. A method in accordance with claim 1, wherein said oil comprises at least 40 % DHA.

14. A method in accordance with claim 1, wherein the oil is at least substantially free of EPA.

15. A method in accordance with claim 1, wherein the DHA is in the form of a triglyceride.

16. A method in accordance with claim 1, wherein said DHA oil is obtained by cultivating at least one microorganism which can be cultivated to produce a DHA-containing oil under DHA oil-producing conditions and recovering the oil product.

17. A method in accordance with claim 16, wherein said microorganism is an alga or a fungus.

18. A method in accordance with claim 17, wherein said alga is a dinoflagellate.

19. A method in accordance with claim 18, wherein said dinoflagellate is *Crypthecodinium.*

20. A method in accordance with claim 17, wherein said fungus is a Chytridomycete.

21. A method in accordance with claim 20, wherein said Chytridomycete comprises a *Thraustochytrium* or a species of *Schitzochytrium.*

22. A method in accordance with claim 17, wherein said fungus is an Oomycete.

23. A method in accordance with claim 22, wherein said Oomycete comprises a species of *Mortierella, Saprolegnia* or *Mucor.*

24. A method in accordance with claim 1, wherein said oil is administered orally.

25. A method in accordance with claim 1, wherein said oil is administered in a capsule, a tablet or an emulsion.

26. A method in accordance with claim 1, wherein said oil is administered parenterally.

27. A method in accordance with claim 1, wherein said oil is administered topically.

28. A method in accordance with claim 1, wherein the microbial oil is administered as a component of a prepared food product.

29. A method in accordance with claim 18, wherein the food product comprises a cooking oil, margarine, shortening, spoonable dressing or liquid dressing.

30. A method of alleviating adverse effects of a neurological disorder **characterized by** a DHA and ARA deficiency-associated pathology which comprises administering to a patient affected by such a disorder a single cell microbial oil comprising DHA and a single cell microbial oil comprising ARA in amounts effective to raise the level of circulating DHA in the patient's blood to within the range of about 10 to 150 µg DHA per ml of plasma and the level of circulating ARA to within the range of about 80 to 500 µg ARA per ml of plasma.

31. A method in accordance with claim 30, wherein the disorder is a neurodegenerative disorder.

32. A method in accordance with claim 30, wherein the disorder is a peroxisomal disorder.

33. A method in accordance with claim 32, wherein the peroxisomal disorder comprises Zellweger's syndrome, neonatal adrenoleukodystrophy, infantile Refsum disease, hyperpepecolic acidemia, Rhizomelic chondrodysplasia punctata, Zellweger-like syndrome, adrenoleukodystrophy, adrenomyeloneuropathy, acyl-CoA oxidase deficiency, bifunctional protein deficiency, thiolase deficiency, hyperoxaluria type I, acatalasaemia or adult Refsum disease.

34. A method in accordance with claim 31, wherein the neurodegenerative disorder is Alzheimer's disease.

35. A method in accordance with claim 31, wherein the neurodegenerative disorder is Huntington's disease.

36. A method in accordance with claim 30, wherein the disorder is schizophrenia.

37. A method in accordance with claim 30, wherein the disorder is diabetic neuropathy.

38. A method in accordance with claim 30, wherein the disorder is a neuropathy induced by the ingestion of pro-oxidant heavy metals selected from the group comprising lead, aluminum and iron.

39. A method in accordance with claim 30, wherein the DHA and ARA oils are administered so as to provide DHA at a dosage level of about 50 mg per day to about 5000 mg per day and to provide ARA at a dosage level of about 50 mg per day to about 5000 mg per day.

40. A method in accordance with claim 39, wherein said DHA oil and said ARA oil are administered so as to provide DHA at a dosage level of about 500 mg per day and to provide ARA at a dosage level of about 1000 mg per day.

41. A method in accordance with claim 30, wherein said DHA oil comprises at least 20 % DHA and said ARA oil comprises at least 10% ARA.

42. A method in accordance with claim 30, wherein said DHA microbial oil comprises at least 40 % DHA and the ARA microbial oil comprises at least 40 % ARA.

43. A method in accordance with claim 30, wherein the oils are at least substantially free of EPA.

44. A method in accordance with the method of claim 30, wherein the DHA and ARA are in the form of triglycerides.

45. A method in accordance with claim 30, wherein said DHA oil is obtained by cultivating at least one alga or fungus which can produce a DHA-containing oil under DHA oil-producing conditions and said ARA oil is obtained by cultivating at least one alga or fungus which can produce an ARA-containing oil under ARA oil-producing conditions and recovering the oils produced.

46. A method in accordance with claim 45, wherein said DHA oil-producing algae is a dinoflagellate.

47. A method in accordance with claim 46, wherein the dinoflagellate is a species of *Crypthecodinium.*

48. A method in accordance with claim 45, wherein said DHA-producing fungus is a Chytridomycete.

49. A method in accordance with claim 48, wherein said Chytridomycete comprises a species of *Thraustochytrium* or *Schitzochytrium.*

50. A method in accordance with claim 45, wherein said DHA oil-producing fungus is an Oomycete.

51. A method in accordance with claim 50, wherein said Oomycete is selected from the group consisting of *Mortierella, Saprolegnia,* and *Mucor.*

52. A method in accordance with claim 45, wherein said ARA oil-producing fungus comprises a species of *Pythium* or *Mortierella.*

53. A method in accordance with claim 45, wherein said ARA oil-producing fungus comprises *Pythium insidiosum* or *Mortierella alpina.*

54. A method in accordance with claim 45, wherein said ARA oil-producing algae comprises a species of *Ochromonas, Euglena* or *Porphyridium.*

55. A method in accordance with claim 30, wherein said oil is administered orally.

56. A method in accordance with claim 30, wherein said oil is administered in a capsule, a tablet or an emulsion.

57. A method in accordance with claim 30, wherein said oil is administered parenterally.

58. A method in accordance with claim 30, wherein said oil is administered topically.

59. A method in accordance with claim 30, wherein the microbial oil is administered as a component of a prepared food product.

60. A method in accordance with claim 59, wherein the food product comprises a cooking oil, margarine, shortening, spoonable dressing or liquid dressing.

61. A method of alleviating adverse effects of a neurological disorder **characterized by** an ARA deficiency-associated pathology which comprises administering to a patient affected by such a disorder a single cell microbial oil comprising ARA in an amount effective to raise the level of circulating ARA in the patient's blood to within the range of about 80 to 500 *µg* ARA per ml of plasma.

62. A method in accordance with claim 61, wherein the ARA oil is administered so as to provide ARA at a dosage level of about 50 mg per day to about 5000 mg per day.

63. A method in accordance with claim 62, wherein the ARA oil is administered so as to provide ARA at a dosage level of about 1000 mg per day.

64. A method in accordance with claim 61, wherein said ARA oil comprises at least 10% ARA.

65. A method in accordance with claim 61, wherein the oil is at least substantially free of EPA.

66. A method in accordance with claim 61, wherein the ARA is in the form of a triglyceride.

67. A method of lowering triglyceride content in plasma of a patient which comprises administering to a patient an oil enriched in DHA in an amount effective to lower the level of plasma triglycerides in the patient.

68. The method of lowering triglyceride content in plasma of a patient according to claim 67, wherein the oil enriched in DHA is administered in an amount effective to raise the level of circulating DHA in the patient's blood to at least about 50 % above the normal level of plasma DHA.

69. The method of claim 67, wherein the amount of oil enriched in DHA is effective to raise the level of circulating DHA to within the range of about 15 to 100 µg of DHA per ml of plasma.

70. A method of increasing the level of highly unsaturated fatty acid (HUFA) residues in one or more tissues of a patient having a disease **characterized by** a HUFA deficiency-associated pathology, which comprises administering to a patient a therapeutic composition containing oil comprising HUFA residues in an amount effective to raise the level of HUFA residues in the patient's tissue.

71. The method of claim 70, wherein the therapeutic composition is administered in an amount effective to raise the level of HUFA residues in the patient's tissue to normal.

72. The method of claim 70, wherein the HUFA is DHA.

73. The method of claim 70 or 72, wherein the disease **characterized by** a HUFA deficiency-associated pathology is a neurological disorder.

74. The method of claim 73, wherein the DHA level is normalized in neuronal or brain tissue.

75. A method in accordance with claim 73, wherein the disorder is a peroxisomal disorder.

76. A method in accordance with claim 75, wherein the disorder is a peroxisomal disorder selected from the group consisting of Zellweger's syndrome, neonatal adrenoleukodystrophy, infantile Refsum disease, hyperpepecolic acidemia, Rhizomelic chondrodysplasia punctata, Zellweger-like syndrome, adrenoleukodystrophy, adrenomyeloneuropathy, acyl-CoA oxidase deficiency, bifunctional protein deficiency, thiolase deficiency, hyperoxaluria type I, acatalasaemia and adult Refsum disease.

77. A method in accordance with claim 70 or 72, wherein the disorder is selected from the group consisting of Alzheimer's disease, Huntington's disease, schizophrenia, diabetic neuropathy, and heavy metal toxicity.

78. The method of claim 70 or 72, wherein the disease **characterized by** a HUFA deficiency-associated pathology is selected from the group consisting of multiple sclerosis, cerebral palsy, amyotrophic lateral sclerosis, phenylketonuria and cystic fibrosis.

79. A method for correcting lipid imbalance in a patient having an abnormal lipid profile by supplying to the patient a lipid supplement comprising highly unsaturated fatty acids (HUFA), said method comprising the steps of:
(a) computing a deviation for each HUFA in the patient having abnormal lipid profile, said deviation being the difference between normal tissue level of each HUFA and the level of each HUFA in the tissue of the patient;
(b) preparing a therapeutic supplement by combining a plurality of individual HUFA-containing oils, at least one individual HUFA-containing oil containing one HUFA and being substantially free of all other HUFAs, the individual oils being combined in proportion such that the amount of each HUFA in the therapeutic supplement is approximately proportional to its deviation as calculated in step (a); and
(c) administering an effective amount of the therapeutic supplement to the patient to restore normal tissue lipid profile to the patient.

80. The method for correcting lipid imbalance in a patient having abnormal lipid profile according to claim 79, wherein the tissue is blood.

81. The method for correcting lipid imbalance in a patient having abnormal lipid profile according to claim 80, wherein administration of the therapeutic supplement does not raise the level of each HUFA in the patient's serum above the normal serum level.

82. The method for correcting lipid imbalance in a patient having abnormal serum lipid profile according to claim 79, wherein one of the plurality of individual HUFA-containing oils is a fish oil.

83. A method of enhancing the ability of excitable cell membranes to sustain a chemical gradient of calcium concentration across the cell membranes comprising treating the cells with oil enriched in DHA in an amount effective to raise the level of DHA residues in the cell membranes.

84. The method of claim 83, wherein the cell is a neurological cell or a cardiac muscle cell.
